(19) Europäisches Patentamt
European Patent Office
Office européen des brevets

(11) EP 2 586 871 A1

(12) **EUROPEAN PATENT APPLICATION**
published in accordance with Art. 153(4) EPC

(43) Date of publication:
**01.05.2013 Bulletin 2013/18**

(21) Application number: **11797897.3**

(22) Date of filing: **11.04.2011**

(51) Int Cl.:
***C12P 19/02*** *(2006.01)* ***C13K 1/02*** *(2006.01)*
***C07H 3/02*** *(2006.01)*

(86) International application number:
**PCT/JP2011/058963**

(87) International publication number:
**WO 2011/162009 (29.12.2011 Gazette 2011/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2010 JP 2010143462**

(71) Applicant: **Toray Industries, Inc.**
**Tokyo 103-8666 (JP)**

(72) Inventors:
- **HANAKAWA, Masayuki**
**Otsu-shi**
**Shiga 520-8558 (JP)**
- **HIROZAWA, Hiroho**
**Otsu-shi**
**Shiga 520-8558 (JP)**

(74) Representative: **Hoefer & Partner**
**Pilgersheimer Straße 20**
**81543 München (DE)**

# (54) PROCESS FOR PRODUCTION OF AQUEOUS REFINED SUGAR SOLUTION

(57) Disclosed is a method for producing an aqueous refined sugar solution, in which a biomass residues that are formed during a step of producing a sugar from a cellulose-containing biomass is removed in a step of producing an aqueous sugar solution, thereby preventing the clogging of a microfiltration membrane and/or an ultrafiltration membrane and ensuring long-term, stable production of an aqueous refined sugar solution. Specifically disclosed is a process for producing an aqueous refined sugar solution using a cellulose-containing biomass as a raw material, which comprises the steps of: (1) decomposing cellulose-containing biomass to produce an aqueous sugar solution; (2) subjecting the aqueous sugar solution resulting from step (1) to aggregation treatment with an aggregation agent to produce an aqueous sugar solution having an average particle diameter of 2 μm or more as determined by particle size distribution measurement; and (3) filtering the aqueous sugar solution resulting from step (2) through a microfiltration membrane and/or an ultrafiltration membrane and subsequently recovering an aqueous sugar solution from the downstream side.

Figure 1

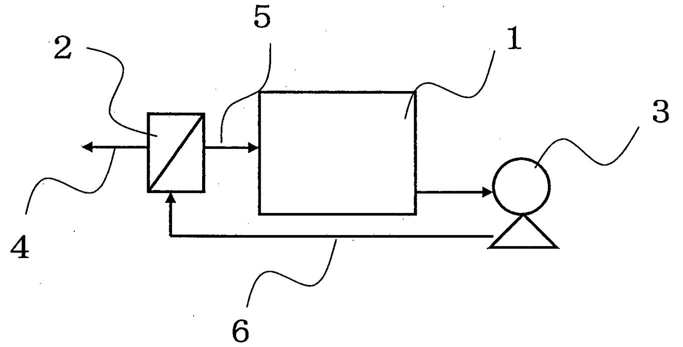


EP 2 586 871 A1

## Description

Technical field

The present invention relates to a method for producing an aqueous refined sugar solution from cellulose-containing biomass.

Background art

After the end of the 20th century, which was a period of mass consumption and mass disposal, construction of an environment-conscious society is now called for in the 21 st century, and with the worsening of the fossil resources depletion and the global warming problem, expectations are growing for the promotion of effective use of biomass as recycled resources.

Among other biomass resources, production of bioethanol from sugar cane and corn is currently performed actively in the U.S. and Brazil. This is because sugar cane and corn are rich in sucrose and starch, and it is easy to prepare an aqueous sugar solution from them and ferment it. However, sugar cane and corn themselves are food, and their use as raw material can cause a serious problem of competition with other foodstuffs and feed materials, leading to a rise in their prices. It is a pressing issue to construct processes for efficiently producing an aqueous sugar solution from nonfood biomass such as cellulose-containing biomass and efficiently using the resulting aqueous sugar solution as fermentation feedstock for conversion into industrial material.

To produce an aqueous sugar solution from cellulose-containing biomass, some aqueous sugar solution production methods that use sulfuric acid have been disclosed such as using concentrated sulfuric acid for acid hydrolysis of cellulose and hemicellulose to produce an aqueous sugar solution (Patent documents 1 and 2), and subjecting cellulose-containing biomass to hydrolysis treatment with dilute sulfuric acid followed by enzyme treatment with, for instance, cellulase to produce an aqueous sugar solution (Non-patent document 1).

There are acid-free methods disclosed so far including use of subcritical water of about 250°C to 500°C to hydrolyze cellulose-containing biomass to produce an aqueous sugar solution (Patent document 3), treatment of cellulose-containing biomass with subcritical water followed by enzyme treatment to produce an aqueous sugar solution (Patent document 4), and hydrolysis treatment of cellulose-containing biomass with compressed hot water of 240°C to 280°C followed by enzyme treatment to produce an aqueous sugar solution (Patent document 5).

However, the resulting aqueous sugar solution contains a large amount of biomass residues, and accordingly, appropriate solid-liquid separation treatment for removal of the biomass residues should be carried out to allow the aqueous sugar solution to serve as fermentation feedstock after being supplied to a fermentation tank.

As a method to remove biomass residues in this way, a filtration method using an ultrafiltration membrane (Patent document 6) has been disclosed, long term stable production of an aqueous sugar solution is difficult due to clogging of the ultrafiltration membrane. A combined use of an ultrafiltration membrane with a nonwoven fabric filter with a pore size of 20 to 200 μm has been disclosed (Patent document 7), but this method cannot prevent ultrafiltration membranes from being clogged biomass residues such as fine particles, turbid substances, and colloid with a size of 20 um or less, though effective for removing biomass residues of about 20 μm or more, failing to perform long term stable, continuous production of an aqueous sugar solution.

Prior art documents

Patent documents

Patent document 1: Japanese Unexamined Patent Publication (Kokai) No. HEI 11-506934
Patent document 2: Japanese Unexamined Patent Publication (Kokai) No. 2005-229821
Patent document 3: Japanese Unexamined Patent Publication (Kokai) No. 2003-212888
Patent document 4: Japanese Unexamined Patent Publication (Kokai) No. 2001-95597
Patent document 5: Japanese Patent No. 3041380
Patent document 6: Japanese Unexamined Patent Publication (Kokai) No. 2006-88136
Patent document 7: Japanese Unexamined Patent Publication (Kokai) No. 2009-240167

[Non-patent documents]

Patent document 1: A. Aden et al., "Lignocellulosic Biomass to Ethanol Process Design and Economics Utilizing

Co-Current Dilute Acid Prehydrolysis and Enzymatic Hydrolysis for Corn Stover" NREL Technical Report (2002)

Summary of the invention

Problems to be solved by the invention

Thus, to solve the above-mentioned problem, the invention provides a method that allows biomass residues that are formed during production of sugars from cellulose-containing biomass to be removed before performing filtration through a microfiltration membrane and/or ultrafiltration membrane, thereby preventing clogging of the microfiltration membrane and/or ultrafiltration membrane to ensure long term stable production of an aqueous refined sugar solution.

Means of solving the problems

As a result of intensive studies on the above-mentioned problem, the present inventors have found that long term stable production of an aqueous sugar solution by separation and removal of biomass residues from sugar that serves as fermentation feedstock can be achieved by carrying out, during a step of producing sugar from cellulose-containing biomass, coagulation treatment of an aqueous sugar solution to produce an aqueous sugar solution with an average particle diameter of 2 μm or more as determined by particle size distribution measurement, and subsequent microfiltration and/or ultrafiltration. Specifically, the present invention has one of the constitutions [1] to [13] described below.

[1] An aqueous refined sugar solution production method using cellulose-containing biomass as feed material, comprising:

(1) a step of decomposing cellulose-containing biomass to produce an aqueous sugar solution,
(2) a step of subjecting the aqueous sugar solution resulting from step (1) to coagulation treatment with a coagulant to produce an aqueous sugar solution with an average particle diameter of 2 μm or more as determined by particle size distribution measurement.
(3) a step of subjecting the aqueous sugar solution resulting from step (2) to microfiltration and/or ultrafiltration to recover an aqueous sugar solution from the downstream side,

[2] An aqueous refined sugar solution production method as described in item [1] further comprising a step of subjecting the aqueous sugar solution resulting from step (3) to nanofiltration and/or reverse osmosis treatment to recover an aqueous refined sugar solution from the upstream side while removing fermentation impeding substances from the downstream side.

[3] An aqueous refined sugar solution production method as described in either item [1] or [2] wherein a cationic polymer coagulant is used for the coagulation treatment in step (2).

[4] An aqueous refined sugar solution production method as described in any of items [1] to [3] wherein an inorganic coagulant and an organic polymer coagulant are used in combination for coagulation treatment in step (2).

[5] An aqueous refined sugar solution production method as described in any of items [1] to [4] wherein coagulation treatment is performed a plurality of times repeatedly in step (2).

[6] An aqueous refined sugar solution production method as described in item [2] wherein said fermentation impeding substances include one or more selected from the group consisting of an organic acid, a furan based compound, and a phenolic compound.

[7] An aqueous refined sugar solution production method as described in item [6] wherein said organic acid is either formic acid or acetic acid.

[8] An aqueous refined sugar solution production method as described in item [6] wherein said furan based compound is either hydroxymethyl furfural or furfural.

[9] An aqueous refined sugar solution production method as described in item [6] wherein said phenolic compound is vanillin, acetovanillin, or syringic acid.

[10] An aqueous refined sugar solution production method as described in any of items [12] to [9] wherein the aqueous sugar solution resulting from said step (2) is an aqueous sugar solution consisting mainly of monosaccharides.

[11] An aqueous refined sugar solution production method as described in item [2] wherein the step of subjecting the aqueous sugar solution resulting from step (3) to nanofiltration and/or reverse osmosis is carried out by first filtering the aqueous sugar solution through a nanofiltration membrane and subsequently filtering the resulting filtrate through a reverse osmosis membrane.

[12] An aqueous refined sugar solution production method as described in item [2] wherein the functional layers of the filters used for nanofiltration membrane and/or reverse osmosis membrane are formed of polyamide.

[13] An aqueous refined sugar solution production method as described in item [2] wherein the functional layer

of the filter used for nanofiltration is formed mainly of crosslinked piperazine polyamide and contains a component as represented by chemical formula 1.

[Chemical formula 1]

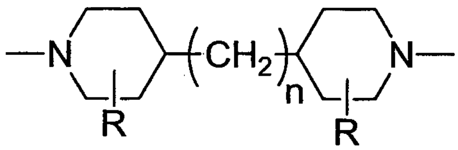

(where R represents -H or $-CH_3$, and n represents an integer of 0 to 3).)

[14] A chemical product production method wherein an aqueous refined sugar solution produced by an aqueous refined sugar solution production method as described in any of items [1] to [13] is used as fermentation feedstock.

Effect of the invention

The present invention serves to remove biomass residues that are formed during the step for producing sugar from cellulose containing biomass so that clogging of the filters used for microfiltration and/or ultrafiltration is prevented, thus ensuring long term stable production of an aqueous refined sugar solution. Accordingly, the use of an aqueous refined sugar solution produced according to the present invention as fermentation feedstock serves to increase the efficiency of fermentative production of various chemical products.

Brief description of the drawings

[Fig. 1] Fig. 1 gives a schematic diagram that schematically shows a filtering apparatus for nanofiltration/reverse osmosis.

Description of embodiments

The invention is described in more detail below.

Cellulose-containing biomass materials used for the aqueous refined sugar solution production method according to the invention include, for instance, herbaceous types such as bagasse, switchgrass, corn stover, rice straw, and wheat straw, and wood based types such as wood and waste building materials. These cellulose-containing biomass materials contain cellulose or hemicellulose, which are polysaccharides resulting from dehydration and condensation of sugars, and an aqueous sugar solution useful as fermentation feedstock can be produced by decomposing these polysaccharides. Here, cellulose-containing biomass to be used for the invention may contain other components unless they interfere with meeting the object of the invention, and for instance, edible biomass components such as sucrose and starch may be contained. When bagasse, which is sugar cane marc, is used as cellulose-containing biomass, for instance, juice of sucrose-containing sugar cane may be used simultaneously.

An aqueous refined sugar solution as referred to for the present invention is an aqueous sugar solution resulting from decomposition of cellulose-containing biomass. For decomposition treatment of cellulose-containing biomass, hydrolysis is preferred as it is simple and low in required cost. Generally, sugars are categorized in terms of the number of monosaccharide constituents into monosaccharides such as glucose and xylose, oligosaccharides resulting from dehydration and condensation of 2 to 9 monosaccharides, and polysaccharides resulting from dehydration and condensation of 10 or more monosaccharides. An aqueous refined sugar solution as referred to for the present invention is an aqueous sugar solution containing a monosaccharide as primary component, and specifically, glucose or xylose is contained as primary component. In addition, oligosaccharides such as cellobiose, and monosaccharides such as arabinose and mannose may also been contained as minor components. For the invention, a monosaccharide as primary component accounts for 80 wt% or more of the total weight of saccharides, including monosaccharides, oligosaccharides, and polysaccharides, dissolved in water. The accurate contents of a monosaccharide, oligosaccharide, or polysaccharide may be analyzed by HPLC (high performance liquid chromatography) and determined quantitatively through comparison with a reference sample. Specific HPLC conditions may be as follows: no reaction liquid used, Luna $NH_2$ column (supplied by Phenomenex), mobile phase with a ratio of ultrapure water : acetonitrile = 25:75, flow rate of 0.6 mL/min, measuring time of 45 min, detection based on RI (differential refractive index), and temperature of 30°C.

Described below is step (1) for subjecting cellulose-containing biomass to decomposition treatment in the

aqueous refined sugar solution production method according to the present invention.

For decomposition treatment of cellulose-containing biomass, unprocessed cellulose-containing biomass may be used, but generally known treatments such as steaming, pulverization, and blasting may also be performed to increase the efficiency of decomposition treatment.

There are no specific limitations on the method to be used for decomposition treatment of cellulose-containing biomass, but specifically, preferred methods include the following six: treatment method A using acid alone, treatment method B using an enzyme after acid treatment, treatment method C using hydrothermal treatment alone, treatment method D using enzyme after hydrothermal treatment, treatment method E using an enzyme after alkali treatment, and treatment method F using an enzyme after ammonia treatment.

Treatment method A performs hydrolysis using an acid for decomposition treatment of cellulose-containing biomass. Usable acids include sulfuric acid, nitric acid, and hydrochloric acid, of which sulfuric acid is preferred.

There are no specific limitations on the acid concentration, and an acid used may account for 0.1 to 99 wt%. When the acid concentration is 0.1 to 15 wt%, preferably 0.5 to 5 wt%, the reaction temperature is set in the range of 100 to 300°C, preferably 120 to 250°C, and the reaction time is set in the range of 1 sec to 60 min. There are no specific limitations on the number of repetition, and the above treatment may be performed once or more. In particular, when the above treatment is performed two or more times, the first and the subsequent treatment runs may be carried out under different conditions.

When the acid concentration is 15 to 95 wt%, preferably 60 to 90 wt%, the reaction temperature is set in the range of 10 to 100°C, and the reaction time is set in the range of 1 sec to 60 min.

There are no specific limitations on repetition of said acid treatment, and the above treatment may be performed once or more. In particular, when the above treatment is performed two or more times, the first and the subsequent treatment runs may be carried out under different conditions.

The hydrolysate resulting from acid treatment contains acids such as sulfuric acid and has to be neutralized before use as fermentation feedstock. There are no specific limitations on the alkali reagent to be used for neutralization, but preferably, a monovalent alkali reagent is used. If both the acid and alkali components are in the form of a di- or higher-valent salt in a case where a filter for nanofiltration is used, they will not pass the filter for nanofiltration, and the salts may be precipitated in the liquid as it is concentrated, possibly leading to fouling.

There are no specific limitations on the monovalent alkali to be used, and usable ones include ammonia, sodium hydroxide, and potassium hydroxide.

If a di- or higher-valent alkali reagent is used, it will be necessary in some cases that the amounts of acid and alkali be decreased to prevent their salts from being precipitated, or a means of removing the precipitate be provided.

Hydrolysis using acid is characterized by hydrolysis of the hemicellulose component, which is lower in crystallinity, tending to take place in an earlier stage, followed by decomposition of the cellulose component, which is higher in crystallinity. Accordingly, a liquid containing a large content of hemicellulose-derived xylose can be produced by using acid. During acid treatment, biomass solid components resulting from the treatment may be subjected to reaction at a higher pressure and temperature than for the treatment so that cellulose components with high crystallinity are decomposed to produce a liquid with a high content of cellulose-derived glucose. Hydrolysis may be carried out in two stages so that suitable hydrolysis conditions can be set up for hemicellulose and cellulose to increase the decomposition efficiency and sugar yield. The aqueous sugar solution resulting under the first decomposition conditions and the aqueous sugar solution resulting under the second decomposition conditions may be separated to make it possible to produce two aqueous sugar solutions containing hydrolysates with different monosaccharide contents. Specifically, an aqueous sugar solution containing xylose as primary component resulting under first decomposition conditions can be obtained separately from an aqueous sugar solution containing glucose resulting under second decomposition conditions. Thus, if monosaccharide components contained in aqueous sugar solutions are separated, fermentation involving xylose in an aqueous sugar solution as fermentation feedstock can be performed separately from fermentation involving glucose as fermentation feedstock, allowing the most suitable microorganisms to be selected and used for fermentation. However, high-pressure, high-temperature acid treatment may be continued for an extended period of time to produce a mixture of both hemicellulose- and hemicellulose-derived sugars without separating the hemicellulose component and the cellulose component.

Treatment method B subjects the treated liquid resulting from treatment method A to further hydrolysis of cellulose-containing biomass using an enzyme. For treatment method B, it is preferable that the acid concentration is 0.1 to 15 wt%, more preferably 0.5 to 5 wt%. The reaction temperature may be set in the range of 100 to 300°C, preferably 120 to 250°C. The reaction time may be set in the range of 1 sec to 60 min. There are no specific limitations on the number of times of repetition, and the above treatment may be performed once or more. In particular, when the above treatment is performed two or more times, the first and the subsequent treatment runs may be carried out under different conditions.

The hydrolysate resulting from acid treatment contains acids such as sulfuric acid and therefore has to be neutralized for subsequent hydrolysis reaction with an enzyme or for use as fermentation feedsrock. Such neutralization

may be performed similarly to the neutralization by treatment method A.

The above enzyme may be any one with cellulose decomposition activity that is selected from common types of cellulases, but it is preferable to use one which contains either exo-type cellulase or endo-type cellulase that both of them have crystalline cellulose decomposition activity. For this purpose, a cellulase produced by Trichoderma spiecies is highly suitable. Trichoderma spiecies are microorganisms classified as filamentous fungus, which secrete a wide variety of cellulases out of their cells. The cellulase to be used for the present invention is one derived from Trichoderma reesei. As an enzyme to be used for hydrolysis, β-glucosidase, which is a cellobiose-degrading enzyme, may be added to improve the efficiency of glucose production, and it may be used in combination with the above cellulase for hydrolysis. There are no specific limitations on the β-glucosidase to be used, but it is preferable to use Aspergillus-derived one. If these enzymes are used for hydrolysis reaction, it is preferably performed at about pH 3 to 7, more preferably about pH 5. The reaction temperature is preferably 40 to 70°C.

When hydrolysis of cellulose-containing biomass with an enzyme is performed after acid treatment, it is preferable that low-crystallinity hemicellulose is hydrolyzed by acid treatment in a first hydrolysis step, followed by a second hydrolysis step where high-crystallinity cellulose is hydrolyzed with an enzyme. Use of an enzyme in the second hydrolysis step allows the cellulose-containing biomass hydrolysis step to proceed more efficiently. Specifically, hydrolysis of the hemicellulose component and partial decomposition of lignin, both substances being contained mainly in the cellulose-containing biomass, take place in the first hydrolysis step using acid, and the resulting hydrolysate is separated into an acid solution and a cellulose-containing solid component, and then an enzyme is added to hydrolyze the cellulose-containing solid component. The separated and recovered dilute sulfuric acid solution contains xylose, which is a pentose, as primary component, and therefore, an aqueous sugar solution can be isolated by neutralizing the acid solution. Furthermore, a monosaccharide component containing glucose as primary component can be produced from the solid hydrolysis reactant containing cellulose. Here, it may also be effective to mix the aqueous sugar solution resulting from neutralization with the solid component, followed by adding an enzyme to cause hydrolysis.

Treatment method C does not include addition of an acid, but adds water so that cellulose-containing biomass accounts for 0.1 to 50 wt%, followed by treatment at a temperature of 100 to 400°C for 1 sec to 60 min. Treatment under such temperature conditions causes hydrolysis of cellulose and hemicellulose. There are no specific limitations on the number of repetition of this treatment, and it is effective if said treatment is performed once or more. If the above treatment is to be performed two or more times, the first and the subsequent treatment runs may be carried out under different conditions.

Decomposition by hydrothermal treatment is characterized by hydrolysis of the hemicellulose component, which is lower in crystallinity, tending to take place in an earlier stage, followed by decomposition of the cellulose component, which is higher in crystallinity. Accordingly, a liquid containing a large content of hemicellulose-derived xylose can be produced by carrying out hydrothermal treatment. During hydrothermal treatment, biomass solid components resulting from the treatment may be subjected to reaction at a higher pressure and temperature than for said treatment so that cellulose components with high crystallinity are decomposed to produce a liquid with a high content of cellulose-derived glucose. Decomposition may be carried out in two stages so that suitable decomposition conditions can be set up for hemicellulose and cellulose to increase the decomposition efficiency and sugar yield. The aqueous sugar solution resulting from the first decomposition conditions and the aqueous sugar solution resulting from the second decomposition conditions may be separated to make it possible to produce two aqueous sugar solutions containing decomposition products with different monosaccharide contents. Specifically, an aqueous sugar solution containing xylose as primary component resulting under first decomposition conditions can be obtained separately from an aqueous sugar solution containing glucose resulting under second decomposition conditions. Thus, if monosaccharide components contained in aqueous sugar solutions are separated, fermentation involving xylose in an aqueous sugar solution as fermentation feedstock can be performed separately from fermentation involving glucose as fermentation feedstock, allowing the most suitable microorganisms to be identified and used for fermentation.

Treatment method D subjects the treated liquid resulting from treatment method C to further hydrolysis of cellulose-containing biomass using an enzyme.

The same enzyme as that used for treatment method B is used here. For enzyme treatment as well, the same conditions as for treatment method B may be adopted.

When hydrolysis of cellulose-containing biomass with an enzyme is performed after hydrothermal treatment, it is preferable that low-crystallinity hemicellulose is hydrolyzed by hydrothermal treatment in a first decomposition step, followed by a second decomposition step where high-crystallinity cellulose is hydrolyzed with an enzyme. Use of an enzyme in the second decomposition step allows the cellulose-containing biomass decomposition step to proceed more efficiently. Specifically, hydrolysis of the hemicellulose component and partial decomposition of lignin, both substances being contained mainly in the cellulose-containing biomass, take place in the first decomposition steps using hydrothermal treatment, and the resulting hydrolysate is separated into an aqueous solution and a cellulose-containing solid component, followed by adding an enzyme to hydrolyze the cellulose-containing solid component. The separated and recovered aqueous solution contains xylose, which is a pentose, as primary component. Furthermore, a monosaccharide component

containing glucose as primary component can be produced from the solid hydrolysis reactant containing cellulose. Here, it may also be effective to mix the aqueous solution resulting from hydrothermal treatment with the solid component, followed by adding an enzyme to cause hydrolysis.

The alkali to be used in treatment method E is preferably sodium hydroxide or calcium hydroxide. These alkali components added here preferably account for 0.1 to 60 wt% relative to cellulose-containing biomass, and treatment may be performed in the temperature range of 100 to 200°C, preferably 110°C to 180°C. There are no specific limitations on the number of repetition, and the above treatment may be performed once or more. In particular, when the above treatment is performed two or more times, the first and the subsequent treatment runs may be carried out under different conditions.

The treatment product resulting from alkali treatment contains alkalis such as sodium hydroxide and therefore has to be neutralized for subsequent hydrolysis reaction with an enzyme. There are no specific limitations on the acid reagent to be used for neutralization, but preferably, a monovalent acid reagent is used. If both the acid and alkali components are in the form of a di- or higher-valent salt in a case where a filter for nanofiltration is used, they will not pass the filter for nanofiltration, and the salts will be precipitated in the liquid as it is concentrated, leading to fouling.

There are no specific limitations on the monovalent acid to be used, and usable ones include nitric acid and hydrochloric acid.

If a di- or higher-valent acid reagent is used, it will be necessary in some cases that the amounts of acid and alkali be decreased to prevent their salts from being precipitated, or a means of removing the precipitate be provided. If using a di- or higher-valent acid is to be used, it is preferably either sulfuric acid or phosphoric acid.

The same enzyme as that used for treatment method B is used here. For enzyme treatment conditions as well, the same conditions as for treatment method B may be adopted.

When hydrolysis of cellulose-containing biomass with an enzyme is performed after alkali treatment, it is mixed into an aqueous alkali solution and then heated to remove lignin components that are similar to the hemicellulose and cellulose components to allow the hemicellulose and cellulose components to easily undergo reaction, followed by hydrolysis of low-crystallinity hemicellulose and high-crystallinity cellulose components that remain undecomposed by alkali treatment. Specifically, hydrolysis of some hemicellulose components and partial decomposition of lignin, both substances being contained mainly in the cellulose-containing biomass, take place in the alkali treatment, and the resulting hydrolysate is separated into an alkali solution and a cellulose-containing solid component, followed by adjusting the pH value and adding an enzyme to the cellulose-containing solid component to cause hydrolysis. If the concentration of the alkali solution is low, it may be directly subjected to hydrolysis by adding an enzyme after neutralization, without separation of the solid component. Furthermore, a monosaccharide component containing glucose and xylose as primary components can be produced from the solid hydrolysis reactant containing cellulose. The separated and recovered alkali solution contains xylose, which is a pentose, as primary component, in addition to lignin, and therefore, an aqueous sugar solution can be isolated by neutralizing the alkali solution. Here, it may also be effective to mix the aqueous sugar solution resulting from neutralization with the solid component, followed by adding an enzyme to cause hydrolysis.

Treatment method F is carried out under ammonia treatment conditions as specified in Japanese Unexamined Patent Publication (Kokai) No. 2008-161125 and Japanese Unexamined Patent Publication (Kokai) No. 2008-535664. For instance, the ammonia added to cellulose-containing biomass accounts for 0.1 to 15 wt% relative to the cellulose-containing biomass, and treatment is performed at 4°C to 200°C, preferably 90°C to 150°C. The ammonia to be added may be either in a liquid state or in a gas state. When added, furthermore, it may be in the form of either pure ammonia or an aqueous ammonia solution. There are no specific limitations on the number of times of repetition, and the above treatment may be performed once or more. In particular, when the above treatment is performed two or more times, the first and the subsequent treatment runs may be carried out under different conditions.

The treatment product resulting from ammonia treatment should be subjected to neutralization of ammonia or removal of ammonia to allow further hydrolysis reaction to be carried out using an enzyme. There are no specific limitations on the acid reagent to be used for neutralization. For instance, hydrochloric acid, nitric acid, or sulfuric acid may be used, of which sulfuric acid is more preferable because it does not work to corrode the process piping or act as a fermentation impeding factor. Ammonia can be removed by maintaining the ammonia treatment product under reduced pressure to volatilize the ammonia in a gas state. The ammonia removed may be recovered for recycled use.

For enzyme-catalyzed hydrolysis following ammonia treatment, it is known that the crystal structure of cellulose commonly undergo changes caused by ammonia treatment into a crystal structure liable to enzyme reaction. Accordingly, hydrolysis can be achieved efficiently by allowing an enzyme to react with the solid component resulting from such ammonia treatment. The same enzyme as that used for treatment method B is used here. For enzyme treatment conditions as well, the same conditions as for treatment method B may be adopted.

The use of an aqueous ammonia solution can serve to allow the water component, in addition to ammonia, to undergo treatment method C (hydrothermal treatment) during the ammonia treatment, possibly causing hydrolysis of hemicellulose and decomposition of lignin. When hydrolysis of cellulose-containing biomass with an enzyme is performed after treatment with an aqueous ammonia solution, it is mixed into an aqueous ammonia solution and then heated to

remove lignin components that are around the hemicellulose and cellulose components to allow the hemicellulose and cellulose components to easily undergo reaction, followed by hydrolysis with enzyme of low-crystallinity hemicellulose and high-crystallinity cellulose components that remain undecomposed under hydrothermal conditions during the ammonia treatment. Specifically, hydrolysis of some hemicellulose components and partial decomposition of lignin, both substances being contained mainly in the cellulose-containing biomass, take place in the treatment with an aqueous ammonia solution, and the resulting hydrolysate is separated into an aqueous ammonia solution and a cellulose-containing solid component, followed by adjusting the pH value and adding an enzyme to the cellulose-containing solid component to cause hydrolysis. When the ammonia concentration is as high as nearly 100%, a large part of the ammonia may be removed by deaeration, followed immediately by neutralization and addition of an enzyme to cause hydrolysis, without separation of the solid component. Furthermore, a monosaccharide component containing glucose and xylose as primary components can be produced from the solid hydrolysis reactant containing cellulose. The separated and recovered aqueous ammonia solution contains xylose, which is a pentose, as primary component, in addition to lignin, and therefore, an aqueous sugar solution can be isolated by neutralizing the alkali solution. Here, it may also be effective to mix the aqueous sugar solution resulting from neutralization with the solid component, followed by adding an enzyme to cause hydrolysis.

The aqueous sugar solution resulting from step (1) contains not only sugar but also biomass residues such as colloids, turbid substances, and fine particles. The constituents of such biomass residues contain include, but not limited to, lignin, tannin, silica, calcium, and undecomposed cellulose.

Removal of these biomass residues is necessary to allow the aqueous sugar solution resulting from step (1) to work as fermentation feedstock after being supplied to a fermentation tank, and in this regard, the present application inventors made intensive studies using filters for microfiltration membrane and/or ultrafiltration membrane. As a result, it was found that mainly biomass residues with a particle diameter of 1 μm or less caused fouling on the filters for microfiltration and/or ultrafiltration during their continuous operation to prevent long term stable filtration.

Then, the present application inventors found that fouling from biomass residues with a particle diameter of 1 μm or less can be prevented by subjecting the biomass residues to coagulation treatment with a coagulant to produce an aqueous sugar solution in a specific state and supplying it to microfiltration and/or ultrafiltration steps.

Thus, they developed the aqueous refined sugar solution production method according to the present invention, which includes: step (1) for decomposing cellulose-containing biomass to produce an aqueous sugar solution, step (2) for coagulating the aqueous sugar solution resulting from (1) with a coagulant to produce an aqueous sugar solution having an average particle diameter of 2 μm or more as determined by particle size distribution measurement, and step (3) for subjecting the aqueous sugar solution resulting from step (2) to microfiltration membrane and/or ultrafiltration membrane to recover an aqueous sugar solution from the downstream side.

Described below is step (2) of the aqueous refined sugar solution production method according to a step of the present invention, where the aqueous sugar solution resulting from step (1) is subjected to coagulation treatment with a coagulant to produce an aqueous sugar solution with an average particle diameter of 2 μm or more as determined by particle size distribution measurement.

Since as described above, biomass residues with a particle diameter 1 μm or less, such as colloids, turbid substances, and fine particles, contained in the aqueous sugar solution can cause fouling on the filters, it is necessary to remove or reduce them before feeding the solution to microfiltration and/or ultrafiltration. In cases where an aqueous sugar solution is handled in small amounts of about several liters or less, an ultracentrifuge operable at a gravity acceleration of a few tens of thousands of G's can effectively work to remove or reduce them, and therefore, there used to be no significant problems encountered in the laboratory. A common ultracentrifuge, however, is low in capacity and cannot process an aqueous sugar solution in large batches of several tens of liters or more. For long-term, stable production of an aqueous refined sugar solution as intended by the present invention, therefore, there has been a call for a different type of equipment that can remove or reduce biomass residues.

The present application inventors found that even for a large quantity of aqueous sugar solution, clogging of filters was prevented during microfiltration and/or ultrafiltration by performing coagulation treatment with a coagulant to coagulate and precipitate biomass residues, thereby producing an aqueous sugar solution with an average particle diameter of 2 μm or more as determined by particle size distribution measurement. It is preferable that the above coagulation treatment with a coagulant to coagulate and precipitate biomass residues is performed so that the resulting aqueous sugar solution will have an average particle diameter of 3 μm or more as determined by particle size distribution measurement, because it serves more effectively to prevent fouling on the filters used for microfiltration and/or ultrafiltration. For the present invention, the term "coagulation treatment" refers to removal or reduction of biomass residues, including colloids, turbidity components, and fine particles, contained in an aqueous sugar solution.

There are many unclear points in the phenomenon of coagulation and precipitation of biomass residues contained in an aqueous sugar solution performed with a coagulant, but it is inferred as follows. Biomass residues contained in an aqueous sugar solution commonly have negatively charged surfaces, and this negative charge causes the biomass components to repel each other, thereby preventing them from coming into contact with each other and allowing them

to disperse stably in the aqueous sugar solution. If a coagulant having the opposite charge to them is added here, the charge on the surfaces of the biomass residues will be neutralized to cause a decrease in their repulsive force, and they will come closer to each other due to transportation by Brownian movement and water flows, resulting in their bonding to each other to form agglomerated particles. Furthermore, these agglomerated particles grow in size as a result of repeated mutual collision and agglomeration, and the bonds between the agglomerated particles are strengthened by the crosslinking action of the coagulant, result in in the formation of coagulated flocs and their precipitation.

There are no specific limitations on the coagulant to be used for the invention as long as it can serve for coagulation and precipitation of biomass residues contained in an aqueous sugar solution to form an aqueous sugar solution with an average particle diameter 2 μm or more as determined by particle size distribution measurement, but useful inorganic coagulants include, for instance, aluminum salts such as aluminum sulfate, polychloride aluminum, ammonium alum, and potash alum; iron salts such as ferric chloride, ferrous sulfate, and ferric sulfate; and others such as polysilicate-iron. Useful organic polymer coagulants include cationic polymer coagulants, anionic polymer coagulants, nonionic polymer coagulants, and catanionic polymer coagulants, and more specifically, they include acrylamide based anionic polymer, acrylamide based nonionic polymer, acrylamide based cationic polymer, acrylamide based catanionic polymer, acrylic cationic polymer, acrylamide/acrylic acid copolymer, acrylamide/acrylamide-2-methyl propane sulfonic acid copolymer, polyalkyl aminomethacrylate, polyamidine hydrochloride, quaternary ammonium salt polymer, and chitosan. Biomass residues contained in an aqueous sugar solution are small in particle size and they are easily charged negatively. Accordingly, to produce an aqueous sugar solution with an average particle diameter 2 μm or more as determined by particle size distribution measurement, it is preferable that the coagulant to be used can act not only for neutralization of electric charges but also crosslink formation. Cationic polymer coagulants are particularly preferred, and quaternary ammonium salt polymers are highly preferred. Inorganic coagulants and organic polymer coagulants as listed above may be used in combination, and such combinations can serve for effective coagulation treatment in some cases.

There are no specific limitations on the concentration of a coagulant added for coagulation treatment as long as it serves effectively for removing or reducing biomass residues, including colloid components, turbidity components, and fine particles, that are contained in an aqueous sugar solution, but required treatment cost can increase with an increasing concentration and the cost can also increase if the concentration is so low that it becomes difficult to produce an aqueous sugar solution with an average particle diameter 2 μm or more as determined by particle size distribution measurement and that a lengthy still-standing time will be needed after the rapid stirring and slow stirring steps which will be described later. Accordingly, the coagulant preferably accounts for 1,000 to 20,000 ppm, more preferably 3,000 to 15,000 ppm, and still more preferably 5,000 to 10,000 ppm.

For the coagulation treatment, other coagulation assistants such as floc formation assistant and pH adjustor may be used in addition to said coagulant. If the above coagulant is high in charge neutralization ability but poor in crosslinking ability and unable to produce agglutinated flocs with a large average particle diameter, it is particularly preferable to use a floc formation assistant in combination with the agent. Examples of the floc formation assistant include active silicic acid and other negatively charged fine colloid substances. Examples of the pH adjustor include inorganic acids such as sulfuric acid, hydrochloric acid, and nitric acid, and inorganic alkalis such as sodium hydroxide, sodium carbonate, sodium hydrogen carbonate, slaked lime, unslaked lime, and ammonia.

After the addition of said coagulant and/or said coagulation assistant, rapid stirring is performed so that the charges on the surfaces of biomass residues are neutralized, leading to formation of small flocs. It is preferable that this rapid stirring is followed by slow stirring to cause these small flocs to hit each other and grow in size so that they will precipitate more easily. After the slow stirring, it is also preferable that the solution is allowed to stand still to allow biomass residues, including colloid components, turbidity components, and fine particles, to be removed in the form of precipitation, followed by separation of the supernatant liquid for use as an aqueous sugar solution. Here, the optimum strength and time of rapid stirring and slow stirring, as well as the still-standing time, vary depending on the quality of the aqueous sugar solution resulting from said step (1), and therefore, they may be determined based on experimental data while taking into consideration of the balance between required cost and quality of treated solution.

Said coagulation step may be carried out in multiple stages for repeated treatment to promote efficient removal of biomass residues, and in such cases there are no specific limitations on said multiple stages and they may be performed under either the same or different coagulation conditions.

Here, the optimum coagulation treatment conditions including the type of coagulant to be used, use/nonuse of a coagulation assistant, and stirring conditions may be determined based on experimental data while taking account the balance between required cost and quality of treated solution and also considering the cost for the entire process for aqueous sugar solution utilization.

For the present invention, the particle size distribution can be measured by using a laser zeta potential analyzer. Examples of the laser zeta potential analyzer include, for instance, ELS-8000 and ELSZ-2 manufactured by Otsuka Electronics Co., Ltd.

Samples used for particle size distribution measurement for the present invention may be prepared or sampled,

for instance, as described below. First, 100 g of an aqueous sugar solution is left to stand for 6 hours. Then, a pipet is put into the aqueous sugar solution to a depth of about 1 cm from the surface to take a sample. Here, if a coagulation-treated aqueous sugar solution is subjected to particle size distribution measurement, it has to be left to stand for several hours during the treatment operation before recovering the supernatant. This still-standing time during the coagulation treatment operation should be included in the above-mentioned 6-hour still-standing time required before taking a sample.

Described below is step (3) of the aqueous refined sugar solution production method according to the present invention, which is designed to subject the aqueous sugar solution resulting from step (2) to microfiltration and/or ultrafiltration and recover an aqueous sugar solution from the downstream side.

The filter to be used for microfiltration for the invention is a membrane with an average pore size of 0.01 μm to 5 mm, which is also called microfiltration membrane or abbreviated as MF filter. The filter to be used for ultrafiltration for the invention is a membrane with a molecular weight cut off of 1,000 to 200,000, which is called ultrafiltration membrane or abbreviated as UF filter. In this respect, since pores in filters for ultrafiltration are so small that it is difficult for an electron microscope to determine the diameters of pores existing on the filter surface, and therefore, a parameter called the molecular weight cut off is generally used as an index to represent the size of those pores, instead of the average pore diameter. With respect to the molecular weight cut off, Hymenological Experiments Series Volume III Artificial Membrane (ed. Membrane Society of Japan, committee members Shoji Kimura, Shinichi Nakao, Haruhiko Oya, and Tsutomu Nakagawa, 1993, pub. Kyoritsu Shuppan Co., Ltd, p.92) describes (in Japanese) "A curb produced by plotting the stopping ratio for a solute on the longitudinal axis vs. its molecular weight on the horizontal axis is called a molecular weight cut off curve. The molecular weight at which the stopping ratio reaches 90% is called the molecular weight cut off of a film." Thus, it is generally known to those skilled in the art as an index to represent the performance of a film for ultrafiltration.

There are no specific limitations on the material of a filter for microfiltration or ultrafiltration as along as biomass residues as described above can be removed, but examples include organic materials such as cellulose, cellulose esters, polysulfone, polyethersulfone, chlorinated polyethylene, polypropylene, polyolefin, polyvinyl alcohol, polymethyl methacrylate, polyvinylidene fluoride, and polytetrafluoroethylene, and inorganic materials such as stainless steel and other metals as well as ceramics. For microfiltration or ultrafiltration, an appropriate filter material may be selected taking into account the hydrolysates properties and running cost, but from the viewpoint of handleability, it is preferably be an organic material, preferable examples including chlorinated polyethylene, polypropylene, polyvinylidene fluoride, polysulfone, and polyethersulfone.

If in particular, the aqueous sugar solution resulting from step (2) is filtered by ultrafiltration, the enzyme used for saccharization can be recovered from the upstream side. This enzyme recovery step is described below. An enzyme suitable for decomposition treatment has a molecular weight in the range of 10,000 to 100,000, and the enzyme can be recovered from the fractions left on the upstream side when using a filter for ultrafiltration with a molecular weight cut off that is effective for stopping the enzyme. For efficient recovery of an enzyme used for decomposition treatment, it is preferable to use a filter for ultrafiltration with a molecular weight cut off 10,000 to 30,000. There are no specific limitations on the type of filter for ultrafiltration, and either a flat membrane or a hollow fiber membrane may be used. The consumption of the enzyme can be reduced by recycling the recovered enzyme for the decomposition treatment performed in step (1). Accordingly, if ultrafiltration of an aqueous sugar solution is adopted, it is preferable that the aqueous sugar solution is treated by microfiltration in advance to remove water-soluble polymers and colloid components, which work more strongly than other biomass residue components to cause fouling on the filter used for ultrafiltration.

With respect to the filtrate operation, a multi-stage filtering step where microfiltration or ultrafiltration is repeated twice or more may be carried out for efficient removal of water-soluble polymers and colloid components, and there are no specific limitations on the material and properties of a filter to be used for the step.

In the case, for instance, where the filtrate from microfiltration is treated further by ultrafiltration, it is possible to remove substances such as colloid components and lignin derived water-soluble polymer components (tannin) of several tens of nanometers or less that cannot be removed by microfiltration, those saccharides that are not fully decomposed to monosaccharides in the decomposition step but remain at partial decomposition levels in the form of oligosaccharides or polysaccharides, and the enzyme used for the sugar decomposition treatment.

The present invention proposes an aqueous refined sugar solution production method including (1) a step for hydrolyzing cellulose-containing biomass to produce an aqueous sugar solution, (2) a step for coagulating the aqueous sugar solution resulting from step (1) using a coagulant to produce an aqueous sugar solution with an average particle diameter 2 μm or more as determined by particle size distribution measurement, and (3) a step for filtering the aqueous sugar solution resulting from step (2) through filters for microfiltration and/or ultrafiltration and recovering an aqueous sugar solution from the downstream side, and it may further include a step for filtering the aqueous sugar solution resulting from step (3) through filters for nanofiltration and/or reverse osmosis and recovering an refined sugar solution from the upstream side while removing fermentation impeding substances from the downstream side.

Described below is the step for subjecting the aqueous sugar solution resulting from step (3) to nanofiltration and/or reverse osmosis to recover an aqueous refined sugar solution from the upstream side while removing fermentation

impeding substances from the downstream side,

For the present invention, fermentation impediment is defined as the phenomenon in which the production output, accumulation rate, or production rate of a chemical product decreases during the production of the chemical product using, as fermentation feedstock, an aqueous sugar solution produced from cellulose-containing biomass containing fermentation impeding substances, as compared with the case where a reagent monosaccharide is used as fermentation feedstock. For the invention, there are no specific limitations on the degree of fermentation impediment as the degree of impediment suffered by microorganisms varies depending on the type and quantity of fermentation impeding substances existing in the saccharified solution and the degree of impediment depends also on the type of the resulting chemical product, i.e., the final product from the process.

The aqueous sugar solution resulting from the above cellulose-containing biomass decomposition treatment method inevitably contains fermentation impeding substances although their quantities and constituents may vary depending on the treatment method used and the type of cellulose-containing biomass fed, but the fermentation impeding substances can be removed by treating the aqueous sugar solution by filtering the aqueous sugar solution through nanofiltration and/or reverse osmosis, followed by recovering and aqueous refined sugar solution from the upstream side while removing fermentation impeding substances from the downstream side. The fermentation impeding substances as referred to here are compounds that result from decomposition treatment of cellulose-containing biomass and act, as described above, to impede the fermentation step where an aqueous refined sugar solution produced by the production method according to the invention is used as feed material, and in particular, those formed in the acid treatment step for cellulose-containing biomass are roughly divided into organic acids, furan based compounds, and phenolic compounds.

Specific examples of said organic acids include acetic acid, formic acid, and levulinic acid. Specific examples of the above furan based compounds include furfural and hydroxymethyl furfural (HMF). These organic acids and furan based compounds are decomposition products from glucose and xylose, which are monosaccharides.

Specific examples of said phenolic compounds include vanillin, acetovanillin, vanillic acid, syringic acid, gallic acid, coniferyl aldehyde, dihydroconiferyl alcohol, hydroquinone, catechol, acetoguaiacone, homovanillic acid, 4-hydroxybenzoic acid, 4-hydroxy-3-methoxyphenyl derivatives (Hibbert's ketones), these compounds being derived from lignin or lignin precursors.

In addition, when materials such as waste building materials and plywood are used as cellulose-containing biomass, components of adhesives and paints used for lumbering may be contained as fermentation impeding substances. The above adhesive components include urea resin, melamine resin, phenol resin, and urea melamine copolymer resin. Fermentation impeding substances derived from these adhesive components include acetic acid, formic acid, and formaldehyde.

An aqueous sugar solution resulting from step (1) described above commonly contains at least one of the above substances as fermentation impeding substance, or two or more may be contained in actual cases. It is noted here that these fermentation impeding substances can be detected and quantitatively determined by common analysis methods including thin layer chromatography, gas chromatography, and high performance liquid chromatography.

A useful filter for nanofiltration for the invention is a so-called nano-filter (nanofiltration membrane, NF film) which is generally defined as a "membrane that permeates monovalent ions but blocks divalent ions." Such a membrane contains minute pores of several nanometers or so, and are mainly used for stopping minute particles, molecules, ions, salts, and the like contained in a water flow.

A useful filter for reverse osmosis for the invention is a so-called RO film which is generally defined as a "membrane that has a demineralization function including monovalent ion removal," and generally considered to contain ultrafine pores from several angstroms to several nanometers. Such filters are mainly used to remove ion components in processes such as seawater desalination and ultrapure water production.

"Filtering by nanofiltration and/or reverse osmosis" as referred to for the invention means passing an aqueous sugar solution and/or its derivatives resulting from decomposition treatment of cellulose-containing biomass through filters for nanofiltration and/or reverse osmosis in order to stop or separate an aqueous sugar solution containing dissolved sugars, particularly monosaccharides such as glucose and xylose, on the upstream side while permeating fermentation impeding substances as permeated water or filtrate.

The performance of filters used for nanofiltration and/or reverse osmosis for the invention can be evaluated on the basis of the permeation rate (%) calculated for pertinent compounds (fermentation impeding substances, monosaccharides, etc.) contained in an aqueous sugar solution. The permeation rate (%) is calculated by equation 1.

$$\text{Permeation rate (\%)} = (\text{concentration of pertinent compound on the downstream side / concentration of pertinent compound in non-permeated solution}) \times 100 \qquad \text{(equation 1)}$$

There are no specific limitations on the analysis technique to be used to determine the concentration of a pertinent compound in equation 1 as long as measurements can be made with high accuracy and reproducibility, but preferable techniques include high performance liquid chromatography and gas chromatography. When the pertinent compound is a monosaccharide, the permeation rate of a filter for nanofiltration and/or reverse osmosis used for the invention is preferably as low as possible, whereas when the pertinent compound is a fermentation impeding substance, the permeation rate is preferably as high as possible.

With respect to the permeability for nanofiltration and reverse osmosis, it is preferable that the permeation flow rate per unit area of the filter is $0.5m^3/m^2$/day or more when a 500 mg/L aqueous sodium chloride solution is filtered under a pressure of 0.3 MPa. The permeation flow rate per unit area of a filter (membrane permeation flux) can be evaluated on the basis of calculations made by equation 2 using measurements of the quantity of the permeated solution, duration of sampling of the permeated solution, and the area of the membrane.

Membrane permeation flux ($m^3/m^2$/day) = quantity of permeated solution / area of membrane / liquid sampling duration (equation 2)

Generally, filters for nanofiltration fall under a larger pore size category compared with reverse osmosis, and therefore, it is expected that when nanofiltration is performed, a larger weight of fermentation impeding substances are permeated and removed as compared with reverse osmosis, but the loss by weight of monosaccharides, which are the target materials, also becomes larger on the downstream side than in the case of reverse osmosis. In particular, this tendency is more noticeable at higher sugar concentrations. When reverse osmosis is performed, on the other hand, the filter used has a smaller pore size, and accordingly it is expected that the weight of removed impeding substances with larger molecular weights will decrease as compared with nanofiltration. Thus, it is preferable that an appropriate filter is selected from those for nanofiltration or reverse osmosis on the basis of the weight of the fermentation impeding substances contained in the aqueous sugar solution resulting from the treatment steps described above and the molecular weights of major fermentation impeding substances. Two or more, instead of one, filters may be selected from those for nanofiltration or reverse osmosis depending on the composition of the aqueous sugar solution, and multiple filters may be used in combination to perform filtration.

In this respect, it has been found that when nanofiltration is adopted for producing an aqueous refined sugar solution, the loss of monosaccharides into the filtrate increases rapidly as the concentration of the refined monosaccharides held on the concentrated liquid side of the nanofiltration membrane rises to a high level. For the case of performing reverse osmosis for refining, on the other hand, it has been revealed that the loss of monosaccharides remains constant at nearly zero even at increased monosaccharide concentrations in the concentrated solution, although the overall performance was higher with nanofiltration than with reverse osmosis from the viewpoint of removal of fermentation impeding substances. It has been found that a large amount of fermentation impeding substances can be removed while depressing the loss of monosaccharides into the filtrate, if refining by nanofiltration, which can remove a larger amount of fermentation impeding substances than reverse osmosis, is continued to a point where the loss of sugars into the filtrate is deemed significant, followed by further refining performed by reverse osmosis, which can concentrate monosaccharides without a loss in spite of a slightly lower efficiency in removing fermentation impeding substances than in the case of nanofiltration. Thus, in the case where filters for nanofiltration or reverse osmosis are used in combination to produce an aqueous refined sugar solution for the invention, there are no specific limitations on their combinations, but it is preferable that the aqueous sugar solution resulting from step (3) is filtered first by nanofiltration, followed by further filtration of the resulting filtrate by reverse osmosis.

For the nanofiltration step of the invention, the filters to be used may be of a polymer material such as a cellulose acetate based polymer, polyamide, polyester, polyimide, and vinyl polymer, but they may be formed of a plurality of materials instead of being formed of a single material. With respect to the membrane structure, a filter to be used may be either an asymmetric membrane having a dense layer on at least one side of the membrane with the size of fine pores gradually increasing in the direction from the dense layer into the interior or towards the opposite side of the membrane, or a composite membrane produced by covering the dense layer of an asymmetric membrane with a very thin functional layer formed of a different material. For instance, the above composite membrane may be one as described in Japanese Unexamined Patent Publication (Kokai) No. SHO 62-201606, which comprises a support film formed of polysulfone combined with a nano-filter having a functional layer of polyamide.

Of these, it is preferable to use a composite membrane comprising a functional layer of polyamide, which has a high potential with a combination of high pressure resistance, high permeability, and high solute removal performance. To maintain durability against operating pressure, high permeability, and rejecting performance, the use of a membrane with a structure consisting of a functional layer of polyamide held by a support formed of a porous film or nonwoven

fabric is suitable. A composite semipermeable membrane comprising a support having a functional layer of crosslinked polyamide that is produced through condensation polymerization reaction of a polyfunctional amine and a polyfunctional acid halide is suitable as polyamide semipermeable membrane.

For nanofiltration membranes comprising a functional layer of polyamide, preferable carboxylic acid monomer components to constitute the polyamide include, for instance, aromatic carboxylic acids such as trimesic acid, benzophenone tetracarboxylic acid, trimellitic acid, pyromellitic acid, isophthalic acid, terephthalic acid, naphthalene dicarboxylic acid, diphenyl carboxylic acid, and pyridine carboxylic acid, of which trimesic acid, isophthalic acid, terephthalic acid, and mixtures thereof are more preferable from the viewpoint of solubility in solvents used for membrane production.

Preferable amine monomer components to constitute said polyamide include primary diamines having an aromatic ring such as m-phenylene diamine, p-phenylene diamine, benzidine, methylene bis-dianiline, 4,4'-diaminobiphenyl ether, dianisidine, 3,3',4-triaminobiphenyl ether, 3,3',4,4'-tetraaminobiphenyl ether, 3,3'-dioxy benzidine, 1,8-naphthalene diamine, m(p)-monomethyl phenylene diamine, 3,3'-monomethyl amino-4,4'-diaminobiphenyl ether, 4,N, N'-(4-aminobenzoyl)-p(m)-phenylene diamine- 2,2'-bis(4-aminophenyl benzoimidazole), 2,2'-bis(4-aminophenyl benzo oxazole), and 2,2'-bis(4-aminophenyl benzothiazole, and secondary diamines such as piperazine, piperidine, and derivative thereof, and in particular, nanofiltration membranes comprising a functional layer of a crosslinked polyamide produced from piperazine or piperidine as a monomer are preferred because they have heat resistance and chemical resistance as well as pressure resistance and durability. It is more preferably a polyamide comprising the above crosslinked piperazine polyamide or crosslinked piperidine polyamide as primary component and containing a component as represented by chemical formula (1), and still more preferably a polyamide comprising crosslinked piperazine polyamide as primary component and containing a component as represented by chemical formula (1). Of the polyamides represented by chemical formula (1), those for which n=3 are preferred. Examples of such a nanofiltration membrane that has a functional layer of a polyamide comprising crosslinked piperazine polyamide as primary component and containing a component as represented by chemical formula (1) include, for instance, the one described in Japanese Unexamined Patent Publication (Kokai) No. SHO 62-201606 and more specifically, UTC60, a crosslinked piperazine polyamide based nanofiltration membrane product supplied by Toray Industries, Inc., which has a functional layer of a polyamide comprising crosslinked piperazine polyamide as primary component and containing a component as represented by chemical formula (1) where n=3.

Filters for nanofiltration in the form of spiral type film elements are generally used, and for the present invention as well, it is preferable to use a spiral type film element for nanofiltration. Preferable examples of nanofiltration elements include, for instance, GEsepa nanofiltration membrane supplied by GE Osmonics, which is a cellulose acetate based nanofiltration membrane, NF99 and NF99HF nanofiltration membrane supplied by Alfa Laval, which uses a functional layer of polyamide, NF-45, NF-90, NF-200, NF-270, and NF-400 nanofiltration membrane supplied by FilmTec Corporation, which uses a functional layer of crosslinked piperazine polyamide, and SU-210, SU-220, SU-600, and SU-610 nanofiltration modules supplied by Toray Industries, Inc., which uses a functional layer of polyamide containing crosslinked piperazine polyamide as primary component, of which more preferable are NF99 and NF99HF nanofiltration membrane supplied by Alfa Laval, which uses a functional layer of polyamide, NF-45, NF-90, NF-200, NF-270, and NF-400 nanofiltration membrane supplied by FilmTec Corporation, which uses a functional layer of crosslinked piperazine polyamide, and SU-210, SU-220, SU-600, and SU-610 nanofiltration modules supplied by Toray Industries, Inc., which uses a functional layer of polyamide containing crosslinked piperazine polyamide as primary component, of which still more preferable is SU-210, SU-220, SU-600, and SU-610 nanofiltration modules supplied by Toray Industries, Inc., which uses a functional layer of polyamide containing crosslinked piperazine polyamide as primary component.

For nanofiltration, it is preferable that the aqueous sugar solution resulting from step (3) is supplied to a nanofiltration membrane at a pressure in the range of 0.1 MPa or more and 8 MPa or less. The filter's permeation rate will decrease if the pressure is less than 0.1 MPa, whereas the filter may suffer from damage if it is more than 8 MPa. If the pressure is 0.5 MPa or more and 6 MPa or less, on the other hand, the film's permeation flux is high enough to allow a sugar solution to be permeated efficiently, and the film will be little liable to damage, suggesting that this range is more preferable, and the range of 1 MPa or more and 4 MPa or less is particularly preferable.

With respect to the material of filters for reverse osmosis to be used for the present invention, preferable examples include a composite membrane having a functional layer of a cellulose acetate based polymer (hereinafter also referred to as cellulose acetate based reverse osmosis membrane) and a composite film having a functional layer of polyamide (hereinafter also referred to as polyamide based reverse osmosis membrane). Here, examples of the cellulose acetate based polymer include organic esters of cellulose such as cellulose acetate, cellulose diacetate, cellulose triacetate, cellulose propionate, and cellulose butyrate, which may be used singly or in the form of a mixture thereof or a mixed ester. Examples of the polyamide include linear polymers and crosslinked polymers produced from aliphatic and/or aromatic diamine as monomer.

Specific examples of filters for reverse osmosis to be used for the invention include, for instance, SU-710, SU-720, SU-720F, SU-710L, SU-720L, SU-720LF, SU-720R, SU-710P, SU-720P, TMG10, TMG20-370, and TMG20-400, which are low pressure type polyamide based reverse osmosis modules, SU-810, SU-820, SU-820L, and SU-820FA,

which are high pressure type ones, and SC-L100R, SC-L200R, SC-1100, SC-1200, SC-2100, SC-2200, SC-3100, SC-3200, SC-8100, and SC-8200, which are cellulose acetate based reverse osmosis membranes, all supplied by Toray Industries, Inc., NTR-759HR, NTR-729HF, NTR-70SWC, ES10-D, ES20-D, ES20-U, ES15-D, ES15-U, and LF10-D supplied by Nitto Denko Corporation, RO98pHt, RO99, HR98PP, and CE4040C-30D supplied by Alfa Laval, GE Sepa supplied by GE, and BW30-4040, TW30-4040, XLE-4040, LP-4040, LE-4040, SW30-4040, and SW30HRLE-404 supplied by FilmTec Corporation.

For the present invention, reverse osmosis membranes made of a polyamide based material are preferred. This is because in the case of cellulose acetate based membranes, some enzyme components, particularly some cellulase components, used in preceding steps can pass through them, leading to decomposition of cellulose, a major membrane material, in the course of long-term operation.

With respect to the structure, membranes of a flat, spiral, or hollow fiber type may be used appropriately.

For reverse osmosis membranes having a functional layer of polyamide, preferable carboxylic acid monomers and amine monomers to constitute the polyamide are as mentioned for nanofiltration membranes having a functional layer of polyamide.

For reverse osmosis, it is preferable that the aqueous sugar solution resulting from step (3) is supplied to a reverse osmosis membrane at a pressure in the range of 1 MPa or more and 8 MPa or less. The filter's permeation rate will decrease if the pressure is less than 1 MPa, whereas the filter may suffer from damage if it is more than 8 MPa. If the filtration pressure is 2 MPa or more and 7 MPa or less, on the other hand, the film's permeation flux is high enough to allow a sugar solution to be permeated efficiently, and the film will be little liable to damage, suggesting that this range is more preferable, and the range of 3 MPa or more and 6 MPa or less is particularly preferable.

Fermentation impeding substances are removed as they are subjected to nanofiltration and/or reverse osmosis. Of the above-mentioned fermentation impeding substances, HMF, furfural, acetic acid, formic acid, levulinic acid, vanillin, acetovanillin, and syringic acid are permeated and removed preferentially. The sugar components contained in the aqueous sugar solution, on the other hand, are stopped and filtered out on the upstream side of the filters for nanofiltration and/or reverse osmosis. The above sugar components are mainly monosaccharides such as glucose and xylose, but also include disaccharides, oligosaccharides, and other sugar components that are not fully decomposed to monosaccharides by the decomposition treatment in step (1).

The aqueous refined sugar solution obtained from the upstream side of the filters for nanofiltration and/or reverse osmosis are lower in the contents of fermentation impeding substances in particular, as compared with their initial contents in the aqueous sugar solution prior to being fed to the nanofiltration and/or reverse osmosis step. The sugar components contained in said aqueous refined sugar solution are those derived from cellulose-containing biomass and virtually the same as the sugar components resulting from the decomposition treatment in step (1). Specifically, an aqueous refined sugar solution produced according to the present invention contains glucose and/or xylose as primary monosaccharide components. The proportion between glucose and xylose varies depending on the conditions for the decomposition treatment in step (1) and therefore, there is no limitation in it for the present invention. Specifically, xylose is the major monosaccharide component when hemicellulose is mainly decomposed by the treatment, while glucose is the major monosaccharide component when the cellulose component alone is separated and subjected to decomposition treatment after the decomposition of hemicellulose. In the case where decomposition of hemicellulose and decomposition of cellulose are not particularly separated, both glucose and xylose will be contained as major monosaccharide components in the resulting solution.

Concentration using a concentration apparatus such as evaporator may be performed before nanofiltration and/or reverse osmosis treatment, or the aqueous refined sugar solution may be filtered by nanofiltration and/or reverse osmosis to increase the concentration, but from the viewpoint of energy consumption required for the concentration, it is preferable to adopt the step of performing filtration by nanofiltration and/or reverse osmosis to increase the concentration of the aqueous refined sugar solution. A membrane to be used for this concentration step is one designed to remove ions and low molecular-weight molecules under, as driving force, a pressure difference that is larger than the osmotic pressure of the liquid being treated, and usable examples include, for instance, a membrane formed of a cellulose based material such as cellulose acetate and a membrane consisting of a microporous support covered with a separation-functional layer of polyamide produced through condensation polymerization of a polyfunctional amine compound and a polyfunctional acid halide. It is also preferable that a low-fouling membrane designed mainly for sewage treatment produced by coating the surface of a separation-functional polyamide layer with a solution of a compound having at least one reactive group that reacts with acid halide groups so that covalent bonds are formed between said reactive group and those acid halide groups remaining on the surface of the separation functional layer is adopted for depressing the contamination, i.e., fouling, on the surfaces of filters for nanofiltration and/or reverse osmosis. In this respect, specific examples of nanofiltration and reverse osmosis membranes to be used for liquid concentration are as listed above for nanofiltration and reverse osmosis.

Described below is a method to produce chemical products by using, as fermentation feedstock, an aqueous refined sugar solution produced by the aqueous refined sugar solution production method according to the present

invention.

Chemical products can be produced by using, as fermentation feedstock, an aqueous refined sugar solution produced according to the present invention. An aqueous refined sugar solution produced according to the present invention contains, as primary components, glucose and/or xylose which serve as a carbon source for growth of microorganisms or cultured cells, but on the other hand, contains only an extremely small amount of fermentation impeding substances including furan compounds, organic acids, and aromatic compounds, thereby working effectively as fermentation feedstock, particularly as carbon source.

Microorganisms or cultured cells used for the chemical product production method according to the present invention include, for instance, yeasts such as baker's yeast used widely in the fermentation industry, colon bacillus, bacteria such as coryneform, filamentous fungus, actinomyces, animals cells, and insect cells. Microorganisms and cells to be used may be those isolated from natural environment or those partly modified by mutation or gene recombination. In particular, since an aqueous sugar solution derived from cellulose-containing biomass contains pentoses such as xylose, it is preferable to use microorganisms with an enhanced metabolic pathway for pentoses.

Preferable culture mediums include nitrogen sources and inorganic salts in addition to aqueous refined sugar solutions, and furthermore, liquid culture mediums containing appropriate organic micronutrients such as amino acid and vitamins may be used as needed. An aqueous refined sugar solution according to the present invention contains, as carbon sources, monosaccharides such as glucose and xylose that are useful for microorganisms, but in some cases, it may be effective to add, as carbon sources, saccharides such as glucose, sucrose, fructose, galactose, and lactos, starch saccharified solutions containing these saccharides, sugar cane molasses, beet molasses, Hi Test molasses, organic acids such as acetic acid, alcohols such as ethanol, or glycerin to provide fermentation feedstock. Useful nitrogen sources include ammonia gas, aqueous ammonia, ammonium salts, urea, nitrates, other auxiliary organic nitrogen sources such as, for instance, oil cakes, hydrolyzed soybean solution, casein decomposition products, other amino acids, vitamins, corn steep liquor, yeast, yeast extract, meat extract, peptides such as peptone, various fermentation fungus bodies, and hydrolysates thereof. Useful inorganic salts include phosphates, magnesium salts, calcium salts, iron salts, and manganese salts, which may be added appropriately.

If microorganisms need specific nutrients for growth, those nutrients may be added in the form of authentic samples or natural products containing them. In addition, an antifoam agent may be added as needed.

Cultivation of microorganisms is performed commonly in the range of pH 4-8 at a temperature of 20-40°C. The pH value of a culture solution is adjusted in advance at a specified value in the range of pH 4-8 using an inorganic or organic acid, alkaline substance, urea, calcium carbonate, ammonia gas, or the like. If it is necessary to increase the oxygen supply rate, it may be carried out by means of, for instance, adding oxygen to air so that the oxygen concentration is maintained at 21% or more, compressing the culture, increasing the stirring speed, or increasing the aeration rate.

To produce a chemical product by using, as fermentation feedstock, an aqueous refined sugar solution prepared by the aqueous refined sugar solution production method according to the present invention, an appropriate fermentation culture method generally known to persons skilled in the art may be adopted, but from the viewpoint of productivity, it is preferable to use the continuous culture method disclosed in International Publication WO 2007/ 097260.

There are no specific limitations on the chemical products to be produced as long as they are substances produced in a culture solution by said microorganisms and cells. Specific examples of the above-mentioned chemical products to be produced include alcohols; organic acids, amino acids, nucleic acids, and others that are generally mass-produced in the fermentation industry. Examples thereof, include, for instance, alcohols such as ethanol, 1,3-propanediol, 1,4-butanediol, and glycerol; organic acids such as acetic acid, L-lactic acid, D-lactic acid, pyruvic acid, succinic acid, malic acid, itaconate, and citric acid; nucleic acids such as inosine, guanosine, and other nucleosides; nucleotides such as inosinic acid and guanylic acid; and diamine compounds such as cadaverine. An aqueous refined sugar solution produced by the aqueous refined sugar solution production method according to the present invention may also be applied to produce substances such as enzymes, antibiotics, and recombinant proteins.

## Examples

The aqueous refined sugar solution production method according to the present invention will be described in more detail below with reference to Examples. It should be understood, however, that the present invention is not construed as being limited thereto.

(Reference example 1) Method for determining average particle diameter by particle size distribution measurement

Particle size distribution measurement was carried out using ELS-8000 supplied by Otsuka Electronics Co., Ltd. , and the arithmetic average particle diameter was calculated from particle size values and scattering intensity values obtained from the resulting scattering intensity distribution histogram.

For Examples and Comparative examples given here, preparation and measurement of samples used for

particle size distribution measurement and their injection into cells were carried out as described below. First, 100 g of an aqueous sugar solution obtained in each step was left to stand for 6 hours. After the still-standing time, a pipet was put into the aqueous sugar solution to a depth of about 1 cm from the surface to take a sample, which was poured slowly to a particle diameter measuring cell attached to the ELS-8000 equipment. Here, if a coagulation-treated aqueous sugar solution is subjected to particle size distribution measurement, it must have undergone still-standing for 6 hours during the operation before recovering the supernatant. The recovered supernatant was poured slowly to a particle diameter measuring cell attached to the ELS-8000 equipment.

(Reference example 2) Analysis method for monosaccharide concentration

The concentration of monosaccharides (glucose and xylose) contained in an aqueous sugar solution produced above was determined from comparison with authentic samples under the HPLC conditions listed below.
Column: Luna NH2 (manufactured by Phenomenex)
Mobile phase: ratio of ultrapure water to acetonitrile = 25 : 75 (flow rate 0.6 ml/min)
Reaction liquid: none
Detection method: RI (differential refractive index)
Temperature:30°C

(Reference example 3) Analysis method for concentration of fermentation impeding substances Furan based fermentation impeding substances (HMF, furfural) and phenolic fermentation impeding substances (vanillin, acetovanillin) contained in an aqueous sugar solution were determined from comparison with authentic samples under the HPLC conditions listed below.
Column: Synergi HidroRP 4.6 mm × 250 mm (manufactured by Phenomenex)
Mobile phase: acetonitrile - 0.1% $H_3PO_4$ (flow rate 1.0 ml/min)
Detection method: UV (283 nm)
Temperature: 40°C

Organic acid based fermentation impeding substances (acetic acid, formic acid) contained in an aqueous sugar solution were quantitatively determined from comparison with authentic samples under the HPLC conditions listed below.
Column: Shim-Pack SPR-H and Shim-Pack CR101H (supplied by Shimadzu Corporation) connected in series
Mobile phase: 5mM p-toluene sulfonic acid (flow rate 0.8 ml/min) Reaction liquid: 5mM p-toluene sulfonic acid, 20mM bis-tris, 0.1mM EDTA·2Na (flow rate 0.8 ml/min)
Detection method: electric conductivity
Temperature: 45°C

(Reference example 4) Measuring method for turbidity

Turbidity of an aqueous sugar solution was determined using an advanced indoor turbidity meter (2100N) manufactured by HACH. Here, since this turbidity meter was designed only for measuring turbidity of 1,000 NTU or less, an aqueous sugar solution was diluted, as needed, with distilled water before making measurements.

(Reference example 5) Decomposition treatment step comprising dilute sulfuric acid treatment and enzyme treatment of cellulose-containing biomass

With respect to the cellulose-containing biomass decomposition treatment in step (1), a cellulose-containing biomass hydrolysis method using 0.1 to 15 wt% dilute sulfuric acid and an enzyme will be illustrated below with reference to Examples.
Rice straw was used as cellulose-containing biomass. The cellulose-containing biomass was immersed in a 1% aqueous sulfuric acid solution and subjected to autoclave (manufactured by Nitto Koatsu Co.) treatment at 150°C for 30 min. After the treatment, solid-liquid separation was carried out to separate an aqueous sulfuric acid solution (hereinafter referred to as dilute sulfuric acid treatment liquid) and cellulose treated with sulfuric acid. Then, the cellulose treated with sulfuric acid and the dilute sulfuric acid treatment liquid was mixed and stirred to provide a liquid with a solid

content of 10 wt%, followed by adding sodium hydroxide to adjust the pH value to about 5. To this liquid mixture, Trichoderma cellulase (supplied by Sigma-Aldrich Japan) and Novozyme 188 (Aspergillus-niger-derived β-glucosidase formulation, supplied by Sigma-Aldrich Japan), which are cellulases, were added and stirred at 50°C for 3 days for hydrolysis reaction to provide an aqueous sugar solution. The aqueous sugar solution had a turbidity of 9,000 NTU and an average particle diameter of 1.0 μm as determined by particle size distribution measurement.

Here, the resulting aqueous sugar solution was subjected to solid-liquid separation by centrifugal separation at 3,000G for analysis of the contents of monosaccharides and fermentation impeding substances contained. Results showed that the contents of the monosaccharides and fermentation impeding substances in the aqueous sugar solution were as given in Table 1.

[Table 1]

| | Reference example 4 | Reference example 5 |
|---|---|---|
| turbidity (ntu) | 9,000 | 10,000 |
| average particle diameter (μm) | 1.0 | 1.1 |
| glucose (g/l) | 25 | 50 |
| xylose (g/l) | 12 | 8 |
| formic acid( g/l) | 0.1 | 0.1 |
| acetic acid (g/l) | 2.4 | 0.5 |
| HMF (mg/l) | 125 | 10 |
| furfural (mg/l) | 875 | 15 |
| vanillin (mg/l) | 90 | 3 |
| acetovanillin (mg/l) | 146 | 13 |

(Reference example 6) Decomposition treatment step comprising hydrothermal treatment and enzyme treatment of cellulose-containing biomass

With respect to the cellulose-containing biomass decomposition treatment in step (1), a cellulose-containing biomass hydrolysis method using hydrothermal treatment and an enzyme will be illustrated with reference to Examples.

Rice straw was used as cellulose-containing biomass. The cellulose-containing biomass was immersed in water and subjected to autoclave (manufactured by Nitto Koatsu Co.) treatment while stirring the liquid at 180°C for 20 min. The pressure during this operation was 10 MPa. After the treatment, the liquid was subjected to solid-liquid separation to separate a treated biomass component and a solution component, thereby providing a solid treated biomass component.

Subsequently, after measuring the water content in the treated biomass component, RO water was added to adjust the solid component content to 15 wt% in terms of absolute-dry treated biomass, and then Trichoderma cellulase (supplied by Sigma-Aldrich Japan) and Novozyme 188 (Aspergillus-niger-derived β-glucosidase formulation, supplied by Sigma-Aldrich Japan), which are cellulases, were added and stirred, at 50°C for 3 days for hydrolysis reaction to provide an aqueous sugar solution. The aqueous sugar solution had a turbidity of 10,000 NTU and an average particle diameter of 1.1 μm as determined by particle size distribution measurement.

Here, the resulting aqueous sugar solution was subjected to solid-liquid separation by centrifugal separation at 3,000G for analysis of the contents of monosaccharides and fermentation impeding substances contained. Results showed that the contents of the monosaccharides and fermentation impeding substances in the aqueous sugar solution were as given in Table 1.

(Reference example 7) Measurement of standard flux and measurement of percent decrease in standard flux

Being different in membrane pore size, the filters used for microfiltration, ultrafiltration, nanofiltration, and reverse osmosis differed in standard membrane filtration conditions, and therefore, the quantity of permeated liquid per unit time and unit membrane area was measured under the following conditions for each filter to determine the standard flux.

For filters for microfiltration and ultrafiltration, distilled water of a temperature 25°C was fed at a pressure of 10 kPa to cause the water to be completely filtered, and the quantity of permeated liquid per unit time and unit membrane

area was measured, followed by calculation according to equation 3.

For filters for nanofiltration, a 500 ppm aqueous sodium chloride solution adjusted to a temperature of 25°C and pH 6.5 was fed at a pressure of 0.35 MPa to subject the water to cross-flow filtration, and the quantity of permeated liquid per unit time and unit membrane area was measured, followed by calculation according to equation 3. Here, the linear speed at the membrane during the cross-flow filtration was adjusted to 30 cm/sec.

For filters for reverse osmosis, a 500 ppm aqueous sodium chloride solution adjusted to a temperature of 25°C and pH 6.5 was fed at a pressure of 0.76 MPa to subject the water to cross-flow filtration, and the quantity of permeated liquid per unit time and unit membrane area was measured, followed by calculation according to equation 3. Here, the linear speed at the membrane during the cross-flow filtration was adjusted to 30 cm/sec.

$$\text{Standard flux} = \text{permeated liquid quantity} / \text{membrane area} / \text{sampling time} \qquad \text{(equation 3)}$$

When filtering an aqueous sugar solution, filters for microfiltration, ultrafiltration, nanofiltration and reverse osmosis suffer from fouling due to water-soluble polymer components, colloid components, turbidity components, and/or fine particles in the aqueous sugar solution, leading to a decrease in standard flux. This decrease in standard flux becomes significant as the total filtration quantity increases.

Accordingly, by comparing the standard flux at a point when 1 liter of an aqueous sugar solution has been filtered with the standard flux at a point when 2 liters of the aqueous sugar solution has been filtered, it is possible to estimate the degree of progress of fouling on the filter. Specifically, calculation by equation 4 gives a percent decrease in standard flux. A smaller percent decrease in standard flux given by equation 4 means that the membrane can continue filtration stably for a longer time.

$$\text{Percent decrease in standard flux (\%)} = (1 - \text{standard flux after 2L filtration} / \text{standard flux after 1L filtration}) \times 100 \qquad \text{(equation 4)}$$

(Reference example 8) Selection of coagulant

Six coagulants (coagulants A to F) listed below were used in Examples and Comparative examples.

Coagulant A: a quaternary ammonium salt polymer, Senkaflock DE-30 (supplied by Senka Corporation)
Coagulant B: a quaternary ammonium salt polymer, Senkaflock DE-40 (supplied by Senka Corporation)
Coagulant C: polyaluminum chloride (supplied by Taki Chemical Co., Ltd.)
Coagulant D: polysilicate-iron PSI-100 (supplied by Suido Kiko Kaisha,LTD.)
Coagulant E: acrylic polymer, Sanfloc C-109P (supplied by Sanyo Chemical Industries Ltd.)
Coagulant F: polymethacrylate, Takifloc C-410 (supplied by Taki Chemical Co., Ltd.)

(Example 1)

An aqueous refined sugar solution was produced by the following three steps.

In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.

In step (2), coagulation treatment was carried out as described below. Specifically, coagulant A was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 420 NTU and an average particle diameter of 3.2 μm as determined by particle size distribution measurement.

In step (3), the aqueous sugar solution resulting from step (2) was fed at a pressure of 200 kPa and a temperature of 25°C to a filter for microfiltration to undergo cross-flow filtration, followed by recovering 10 liters of an aqueous sugar solution from the downstream side. Here, the linear speed of cross-flow filtration at the membrane was adjusted to 30 cm/sec, and then, for determination of the percent decrease in standard flux, the filter used for microfiltration was taken

out when 1 liter of the liquid had been filtered, followed by measuring the standard flux (standard flux after 1L filtration), setting the filter for microfiltration again, taking it out after another 1 liter of filtration of the aqueous sugar solution, and measuring the standard flux (standard flux after 2L filtration). For micro filtration, a specimen was cut out from a flat polyvinylidene fluoride membrane with a nominal pore size of 0.08 μm that was used in Membray (registered trademark) TMR140 microfiltration membrane supplied by Toray Industries, Inc. With this filter for microfiltration, the percent decrease in standard flux was found to be a small 20%, suggesting that the filter would serve for long-term, stable supply of an aqueous sugar solution. In this respect, the resulting aqueous sugar solution had a small turbidity of 1 NTU or less, suggesting that the quatity of turbid substances was reduced.

[Table 2]

| | | Example 1 | Example 2 | Example 3 | Example 4 | Example 5 | Example 6 | Example 7 |
|---|---|---|---|---|---|---|---|---|
| step (1) | | hydrothermal treatment · enzyme treatment | dil. sulfuric acid treatment · enzyme treatment | hydrothermal treatment · enzyme treatment | hydrothermal treatment · enzyme treatment | hydrothermal treatment · enzyme treatment | hydrothermal treatment · enzyme treatment | hydrothermal treatment · enzyme treatment |
| step (2) | | coagulation treatment coagulant A (5000ppm) | coagulation treatment coagulant A (5000ppm) | coagulation treatment coagulant B (10000ppm) | coagulation treatment coagulant C (5000ppm) | coagulation treatment coagulant C (10000ppm) | coagulation treatment coagulant A (5000ppm) + coagulant C (5000ppm) | coagulation treatment coagulant B (5000ppm) |
| step (3) | | microfiltration | microfiltration | microfiltration | microfiltration | microfiltration | microfiltration | ultrafiltration |
| average particle diameter after step (2) (μm) | | 3.2 | 3.2 | 3.6 | 2.3 | 2.6 | 5.0 | 3.6 |
| turbidity (NTU) | During supply to step (2) | 10000 | 9000 | 10000 | 10000 | 10000 | 10000 | 10000 |
| | During supply to step (3) | 420 | 300 | 200 | 1400 | 1100 | 60 | 200 |
| percent decrease in standard flux (%) | microfiltration or ultrafiltration | 20 | 16 | 11 | 29 | 26 | 7 | 16 |

(Example 2)

An aqueous refined sugar solution was produced by the following three steps.

In step (1), the same procedure as described in Reference example 5 except for the absence of centrifugal separation at 3,000G was carried out to produce 20 liters of an aqueous sugar solution containing monosaccharides and fermentation impeding substances as shown in Table 1, which had a turbidity of 9,000 NTU and an average particle diameter of 1.0 μm as determined by particle size distribution measurement.

In step (2), coagulation treatment was carried out as described below. Specifically, coagulant A was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 300 NTU and an average particle diameter of 3.2 μm as determined by particle size distribution measurement.

In step (3), the aqueous sugar solution resulting from step (2) was subjected to microfiltration by the same procedure as in Example 1 to undergo cross-flow filtration. With this filter for microfiltration, the percent decrease in standard flux was found to be a small 16%, suggesting that the filter would serve for long-term, stable supply of an aqueous sugar solution.

(Example 3)

An aqueous refined sugar solution was produced by the following three steps.

In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.

In step (2), coagulation treatment was carried out as described below. Specifically, coagulant B was added to the aqueous sugar solution resulting from step (1) to a content of 10,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 200 NTU and an average particle diameter of 3.6 μm as determined by particle size distribution measurement.

In step (3), the aqueous sugar solution resulting from step (2) was subjected to microfiltration by the same procedure as in Example 1 to undergo cross-flow filtration. With this filter for microfiltration, the percent decrease in standard flux was found to be a small 11 %, suggesting that the filter would serve for long-term, stable supply of an aqueous sugar solution.

(Example 4)

An aqueous refined sugar solution was produced by the following three steps.

In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.

In step (2), coagulation treatment was carried out as described below. Specifically, coagulant C was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 1,400 NTU and an average particle diameter of 2.3 μm as determined by particle size distribution measurement.

In step (3), the aqueous sugar solution resulting from step (2) was subjected to microfiltration by the same procedure as in Example 1 to undergo cross-flow filtration. With this filter for microfiltration, the percent decrease in standard flux was found to be a small 29%, suggesting that the filter would serve for long-term, stable supply of an aqueous sugar solution.

(Example 5)

An aqueous refined sugar solution was produced by the following three steps.

In step (1), the same procedure as described in Reference example 6 except for the absence of centrifugal

separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.

In step (2), coagulation treatment was carried out as described below. Specifically, coagulant C was added to the aqueous sugar solution resulting from step (1) to a content of 10,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 1,100 NTU and an average particle diameter of 2.6 μm as determined by particle size distribution measurement.

In step (3), the aqueous sugar solution resulting from step (2) was subjected to microfiltration by the same procedure as in Example 1 to undergo cross-flow filtration. With this filter for microfiltration, the percent decrease in standard flux was found to be a small 26%, suggesting that the filter would serve for long-term, stable supply of an aqueous sugar solution.

(Example 6)

An aqueous refined sugar solution was produced by the following three steps.

In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.

In step (2), coagulation treatment was carried out as described below. Specifically, coagulant A was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 3 hours and recovering 16 liters of the supernatant. Coagulant C was added again to 16 liters of the resulting supernatant to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 14 liters of the supernatant, i.e., coagulation-treated aqueous sugar solution. The resulting aqueous sugar solution had a turbidity of 60 NTU and an average particle diameter of 5.0 μm as determined by particle size distribution measurement.

In step (3), the aqueous sugar solution resulting from step (2) was subjected to microfiltration by the same procedure as in Example 1 to undergo cross-flow filtration. With this filter for microfiltration, the percent decrease in standard flux was found to be a small 7%, suggesting that the filter would serve for long-term, stable supply of an aqueous sugar solution.

(Example 7)

An aqueous refined sugar solution was produced by the following three steps.

In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.

In step (2), coagulation treatment was carried out as described below. Specifically, coagulant B was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 200 NTU and an average particle diameter of 3.6 μm as determined by particle size distribution measurement.

In step (3), the aqueous sugar solution resulting from step (2) was fed at a pressure of 200 kPa and a temperature of 25°C to a filter for ultrafiltration to undergo cross-flow filtration, followed by recovering 10 liters of an aqueous sugar solution from the downstream side. Here, the linear speed of cross-flow filtration at the membrane was adjusted to 30 cm/sec, and then, for determination of the percent decrease in standard flux, the filter used for ultrafiltration was taken out when 1 liter of the liquid had been filtered, followed by measuring the standard flux (standard flux after 1L filtration), setting the filter for ultrafiltration again, taking out the filter after another 1 liter of filtration of the aqueous sugar solution, and measuring the standard flux (standard flux after 2L filtration). For ultrafiltration, a specimen was cut out from a flat polyethersulfone membrane with a molecular weight cut off of 10,000 Da that was used in Dairy UF10k ultrafiltration filter supplied by Hydranautics. With this filter for ultrafiltration, the percent decrease in standard flux was found to be a

small 16%, suggesting that the filter would serve for long-term, stable supply of an aqueous sugar solution.

(Example 8)

Then, 10 liters of the aqueous sugar solution resulting from step (3), i.e., the aqueous sugar solution recovered by cross-flow filtration by microfiltration, was subjected to nanofiltration at a pressure of 3 MPa and a temperature of 25°C to undergo cross-flow filtration. An aqueous refined sugar solution was recovered from the upstream side while permeated water containing fermentation impeding substances was removed from the downstream side, thereby providing 2.5 liters of an aqueous refined sugar solution. Accordingly, 10 liters of the aqueous sugar solution resulting from step (3) in Example 1 was concentrated 4-fold by this operation for nanofiltration. Here, the linear speed of cross-flow filtration at the membrane was adjusted to 30 cm/sec, and then, for determination of the percent decrease in standard flux, the filter used for nanofiltration was taken out when 1 liter of the liquid had been filtered, followed by measuring the standard flux (standard flux after 1L filtration), setting the filter for nanofiltration again, taking out the filter after another 1 liter of filtration of the aqueous sugar solution, and measuring the standard flux (standard flux after 2L filtration). For this nanofiltration step, a UTC60 nanofiltration filter supplied by Toray Industries, Inc., was used. With this filter for nanofiltration, the percent decrease in standard flux was found to be a small 4%, suggesting that the filter would serve for long-term, stable production of an aqueous refined sugar solution.

The resulting aqueous refined sugar solution was diluted 4-fold with distilled water, and its constituents were compared with those of the aqueous sugar solution resulting from step (3) in Example 1. Table 3 compares the contents of monosaccharides and fermentation impeding substances in the aqueous refined sugar solution resulting from nanofiltration with the contents of monosaccharides and fermentation impeding substances in the aqueous sugar solution resulting from step (3) in Example 1, suggesting that the contents of fermentation impeding substances were reduced by this filter for nanofiltration while maintaining those of monosaccharides.

[Table 3]

| | | Example 8 | Example 9 |
|---|---|---|---|
| step (1) | | hydrothermal treatment / enzyme treatment | hydrothermal treatment / enzyme treatment |
| step (2) | | coagulation treatment coagulant A (500ppm) | coagulation treatment coagulant A (500ppm) |
| step (3) | | microfiltration | microfiltration |
| average particle diameter after step (2) | | 3.2 | 3.2 |
| turbidity (NTU) | during supply to step (2) | 10000 | 10000 |
| | during supply to step (3) | 420 | 420 |
| percent decrease in standard flux (%) | microfiltration or ultrafiltration | 20 | 20 |
| percent decrease in standard flux (%) | nanofiltration or reverse osmosis | 4 | 7 |
| (sugar solution after nanofiltration or reverse osmosis) / (sugar solution from step (3) in Example 1) | glucose | 1.0 | 1.1 |
| | xylose | 1.0 | 1.0 |
| | formic acid | 0.4 | 0.6 |
| | acetic acid | 0.3 | 0.5 |
| | HMF | 0.4 | 0.8 |
| | furfural | 0.2 | 0.7 |
| | vanillin | 0.4 | 0.8 |
| | acetovanillin | 0.1 | 0.7 |

(Example 9)

Then, 10 liters of the aqueous sugar solution resulting from step (3), i.e., the aqueous sugar solution recovered by cross-flow filtration by microfiltration, was subjected to reverse osmosis treatment at a pressure of 3 MPa and a

temperature of 25°C to undergo cross-flow filtration. An aqueous refined sugar solution was recovered from the upstream side while permeated water containing fermentation impeding substances was removed from the downstream side, thereby providing 2.5 liters of an aqueous refined sugar solution. Accordingly, 10 liters of the aqueous sugar solution resulting from step (3) in Example 1 was concentrated 4-fold by this operation for reverse osmosis treatment. Here, the linear speed of cross-flow filtration at the membrane was adjusted to 30 cm/sec, and then, for determination of the percent decrease in standard flux, the filter used for reverse osmosis was taken out when 1 liter of the liquid had been filtered, followed by measuring the standard flux (standard flux after 1L filtration), setting the filter for reverse osmosis again, taking out the filter after another 1 liter of filtration of the aqueous sugar solution, and measuring the standard flux (standard flux after 2L filtration). For reverse osmosis, a specimen was cut out from a polyamide based reverse osmosis membrane that was used in a TMG10 polyamide based reverse osmosis membrane module supplied by Toray Industries, Inc. With this filter for reverse osmosis, the percent decrease in standard flux was found to be a small 7%, suggesting that the filter would serve for long-term, stable production of an aqueous refined sugar solution.

The resulting aqueous refined sugar solution was diluted 4-fold with distilled water, and its constituents were compared with those of the aqueous sugar solution resulting from step (3) in Example 1. Table 3 compares the contents of monosaccharides and fermentation impeding substances in the aqueous refined sugar solution resulting from reverse osmosis treatment with the contents of monosaccharides and fermentation impeding substances in the aqueous sugar solution resulting from step (3) in Example 1, suggesting that the contents of fermentation impeding substances were reduced by this filter for reverse osmosis treatment while maintaining those of monosaccharides.

(Comparative example 1)

Except for using coagulant D instead of coagulant A, the same procedure as in Example 1 was carried out to produce an aqueous refined sugar solution.

In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.

In step (2), coagulation treatment was carried out as described below. Specifically, coagulant D was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 2,800 NTU and an average particle diameter of 1.3 μm as determined by particle size distribution measurement.

In step (3), the aqueous sugar solution resulting from step (2) was subjected to microfiltration by the same procedure as in Example 1 to undergo cross-flow filtration. With this filter for microfiltration, the percent decrease in standard flux was found to be a large 40%, suggesting that the filter would not serve for long-term, stable supply of an aqueous sugar solution.

[Table 4]

| | | Comparative example 1 | Comparative example 2 | Comparative example 3 | Comparative example 4 | Comparative example 5 | Comparative example 6 | Comparative example 7 |
|---|---|---|---|---|---|---|---|---|
| step (1) | | hydrothermal treatment · enzyme treatment | dil. sulfuric acid treatment · enzyme treatment | hydrothermal treatment · enzyme treatment | hydrothermal treatment · enzyme treatment | hydrothermal treatment · enzyme treatment | hydrothermal treatment · enzyme treatment | hydrothermal treatment · enzyme treatment |
| step (2) | | coagulation treatment coagulant D (5000ppm) | coagulation treatment coagulant D (5000ppm) | coagulation treatment coagulant E (5000ppm) | coagulation treatment coagulant F (5000ppm) | none | nonwoven fabric with average pore size of 20 µm | coagulation treatment coagulant A (3000ppm) |
| step (3) | | microfiltration | microfiltration | microfiltration | microfiltration | microfiltration | microfiltration | microfiltration |
| average particle diameter after step (2) (µm) | | 1.3 | 1.4 | 1.8 | 1.2 | 1.1 | 1.0 | 1.8 |
| turbidity (NTU) | during supply to step (2) | 10000 | 9000 | 10000 | 10000 | none | 10000 | 10000 |
| | during supply to step (3) | 2800 | 2500 | 3000 | 3000 | 10000 | 2200 | 2000 |
| percent decrease in standard flux (%) | microfiltration or ultrafiltration | 40 | 39 | 42 | 45 | 65 | 36 | 35 |

(Comparative example 2)

Except for using coagulant D instead of coagulant A, the same procedure as in Example 2 was carried out to produce an aqueous refined sugar solution.
In step (1), the same procedure as described in Reference example 5 except for the absence of centrifugal separation at 3,000G was carried out to produce 20 liters of an aqueous sugar solution containing monosaccharides and fermentation impeding substances as shown in Table 1, which had a turbidity of 9,000 NTU and an average particle diameter of 1.0 μm as determined by particle size distribution measurement.
In step (2), coagulation treatment was carried out as described below. Specifically, coagulant D was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 2500 NTU and an average particle diameter of 1.4 μm as determined by particle size distribution measurement.
In step (3), the aqueous sugar solution resulting from step (2) was subjected to microfiltration by the same procedure as in Example 1 to undergo cross-flow filtration. With this filter for microfiltration, the percent decrease in standard flux was found to be a large 39%, suggesting that the filter would not serve for long-term, stable supply of an aqueous sugar solution.

(Comparative example 3)

Except for using coagulant E instead of coagulant A, the same procedure as in Example 1 was carried out to produce an aqueous refined sugar solution.
In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.
In step (2), coagulation treatment was carried out as described below. Specifically, coagulant E was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 3,000 NTU and an average particle diameter of 1.8 μm as determined by particle size distribution measurement.
In step (3), the aqueous sugar solution resulting from step (2) was subjected to microfiltration by the same procedure as in Example 1 to undergo cross-flow filtration. With this filter for microfiltration, the percent decrease in standard flux was found to be a large 42%, suggesting that the filter would not serve for long-term, stable supply of an aqueous sugar solution.

(Comparative example 4)

Except for using coagulant F instead of coagulant A, the same procedure as in Example 1 was carried out to produce an aqueous refined sugar solution.
In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.
In step (2), coagulation treatment was carried out as described below. Specifically, coagulant F was added to the aqueous sugar solution resulting from step (1) to a content of 5,000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 3,000 NTU and an average particle diameter of 1.2 μm as determined by particle size distribution measurement.
In step (3), the aqueous sugar solution resulting from step (2) was subjected to microfiltration by the same procedure as in Example 1 to undergo cross-flow filtration. With this filter for microfiltration, the percent decrease in standard flux was found to be a large 45%, suggesting that the filter would not serve for long-term, stable supply of an aqueous sugar solution.

(Comparative example 5)

Except that no coagulant was used, the same procedure as in Example 1 was carried out to produce an aqueous refined sugar solution.
In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.
Step (2) was not performed.
In step (3), the aqueous sugar solution resulting from step (1) was subjected to microfiltration by the same procedure as in Example 1 to undergo cross-flow filtration. With this filter for microfiltration, the percent decrease in standard flux was found to be a large 65%, suggesting that the filter would not serve for long-term, stable supply of an aqueous sugar solution.

(Comparative example 6)

Except that a nonwoven fabric with an average pore size 20 μm was used for filtration instead of using coagulant A, the same procedure as in Example 1 was carried out to produce an aqueous refined sugar solution.
In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.
Step (2) was not implemented, and the aqueous sugar solution resulting from step (1) was filtered through a polyester-based nonwoven fabric with an average pore size of 20 μm, namely, HMF-180 (supplied by Japan Vilene Co., Ltd., metsuke (weight per unit surface area) 180 $g/m^2$, air permeability 2 $cc/cm^2/s$), and the resulting filtered aqueous sugar solution was recovered. The resulting aqueous sugar solution had a turbidity of 2,200 NTU and an average particle diameter of 1.0 μm as determined by particle size distribution measurement.
In step (3), the aqueous sugar solution resulting from filtration through a nonwoven fabric with an average pore size of 20 μm was subjected to microfiltration by the same procedure as in Example 1 to undergo cross-flow filtration. With this filter for microfiltration, the percent decrease in standard flux was found to be a large 36%, suggesting that the filter would not serve for long-term, stable supply of an aqueous sugar solution.

(Comparative example 7)

Except for adjusting the content of coagulant A to 3,000 ppm, the same procedure as in Example 1 was carried out to produce an aqueous refined sugar solution.
In step (1), the same procedure as described in Reference example 6 except for the absence of centrifugal separation at 3,000G was carried out to produce 20 liters of an aqueous sugar solution containing monosaccharides and fermentation impeding substances as shown in Table 1, which had a turbidity of 10,000 NTU and an average particle diameter of 1.1 μm as determined by particle size distribution measurement.
In step (2), coagulation treatment was carried out as described below. Specifically, coagulant A was added to the aqueous sugar solution resulting from step (1) to a content of 3000 ppm, and sodium hydroxide was added to adjust the pH value to 7.0. After the pH adjustment, rapid stirring was performed at 150 rpm for 30 min, and then slow stirring was performed at 40 rpm for 30 min, followed by leaving the liquid to stand for 6 hours and recovering 16 liters of the supernatant, i.e., coagulation-treated aqueous sugar solution. The resulting aqueous sugar solution had a turbidity of 2,000 NTU and an average particle diameter of 1.8 μm as determined by particle size distribution measurement.
In step (3), the aqueous sugar solution resulting from step (2) was subjected to microfiltration by the same procedure as in Example 1 to undergo cross-flow filtration. With this filter for microfiltration, the percent decrease in standard flux was found to be a large 35%, suggesting that the filter would not serve for long-term, stable supply of an aqueous sugar solution.
For more detailed description of the chemical product production method using, as fermentation feedstock, a refined sugar solution produced according to the present invention, the production of chemical products such as L-lactic acid, ethanol, cadaverine, D-lactic acid, and succinic acid is illustrated below with reference to Examples. It should be understood, however, that chemical products to be produced by the present invention are not limited thereto.

(Reference example 9) Measuring method for concentration of chemical product
[L-lactic acid, D-lactic acid]
The concentrations of accumulated L-lactic acid and D-lactic acid were confirmed on the basis of the quantity of

lactic acid determined by HPLC.
Column: Shim-Pack SPR-H (supplied by Shimadzu Corporation)
Mobile phase: 5 mM p-toluene sulfonic acid (flow rate 0.8 ml/min)
Reaction liquid: 5 mM p-toluene sulfonic acid, 20 mM bis-tris, 0.1 mM EDTA·2Na (flow rate 0.8 ml/min)
Detection method: electric conductivity
Temperature: 45°C
[Ethanol]
The concentration of accumulated ethanol was determined by gas chromatography. Shimadzu GC-2010 Capillary GC TC-1 (GL science) with a size of 15 meter L.* 0.53 mm I.D. and df 1.5 μm was used along with a flame ionization detector to perform detection, followed by calculation and evaluation.
[Cadaverine]
For cadaverine, evaluation was performed by HPLC as described below.
Column used: Capcell Pak C18 (Shiseido Co., Ltd.)
Mobile phase: ratio of 0.1 % (w/w) aqueous phosphoric acid solution vs. acetonitrile = 4.5:5.5 Detection: UV 360 nm
Sample pre-treatment: A 25 μl volume of a sample is mixed with, as internal standard substances, 25 μl of 1,4-diaminobutane (0.03M), 150 μl of sodium hydrogen carbonate (0.075M), and an ethanol solution of 2,4-dinitro-fluorobenzene (0.2M), and stored at a temperature of 37°C for 1 hour.
A 50 μl portion of the above reaction solution was dissolved in 1 ml of acetonitrile, and subjected to centrifugal separation at 10,000 rpm for 5 min, followed by HPLC analysis for 10 μl of the supernatant.

[Succinic acid]

The concentration of accumulated succinic acid was measured by HPLC (LC10A supplied by Shimadzu Corporation, RID-10A RI monitor, Aminex HPX-87H column). The column was maintained at a temperature of 50°C and equilibrated with 0.01N $H_2SO_4$, followed by injecting a sample and eluting it with 0.01N $H_2SO_4$ for analysis.

(Reference example 10) L-lactic acid fermentation

An L-lactic acid bacteria fermentation media as shown in Table 5 was used, and high pressure steam sterilization (121°C, 15 min) was carried out. The lactobacillus species used was Lactococcus lactis JCM7638, which is a prokaryotic microorganism, and the medium used for lactic acid fermentation by lactobacillus had a composition as shown in Table 5. The L-lactic acid contained in the fermentation liquor was evaluated in the same manner as for Reference example 2. Glucose Test Wako C (supplied by Wako Pure Chemical Industries, Ltd.) was used for measuring the glucose concentration.
Lactococcus lactis JCM7638 was cultured stationarily at a temperature of 37°C for 24 hours in a lactic acid fermentation culture medium in a test tube purged with 5 ml of nitrogen gas (preculture). The resulting culture solution was put in a 50 ml fresh lactic acid fermentation culture medium purged with nitrogen gas, and cultured at a temperature of 37°C for 48 hours (main culture).
[Table 5]

| constituent | glucose | yeast extract | polypeptone | sodium chloride |
|---|---|---|---|---|
| content of constituent | 50 g/L | 5 g/L | 5 g/L | 5 g/L |

(Reference example 11) Ethanol fermentation

Ethanol fermentation by a yeast strain (OC2, Saccharomyces cerevisiae, wine yeast) was studied. The culture medium consisted of glucose as carbon source and the other components of Yeast Synthetic Drop-out Medium Supplement Without Tryptophan (Sigma-Aldrich Japan, Table-6 Drop-out MX), Yeast Nitrogen Base w/o Amino Acids and Ammonium Sulfate (Difco, Yeast NTbase) and ammonium sulfate, which were mixed at a ratio given in Table 6. The culture medium was filter-sterilized (Millipore, Stericup 0.22 μm) before use for fermentation. The glucose concentration was determined using Glucose Test Wako (supplied by Wako Pure Chemical Industries, Ltd.). The quantity ethanol produced in each culture solution was measured by gas chromatography. The OC2 strain was put in a 5ml fermentation culture medium (preculture culture medium) in a test tube and shaken overnight for culture (preculture). From the preculture solution, yeast was recovered by centrifugal separation and rinsed adequately with 15 ml of sterilized water. The yeast rinsed was put in each of 100 ml culture mediums with a composition as shown in Table 6, and shaken for

24 hours in a 500 ml Sakaguchi flask to ensure culture (main culture).

[Table 6]

| constituent | glucose | Drop-out MX | Yeast NTbase | ammonium sulfate |
|---|---|---|---|---|
| content of constituent | 50 g/L | 3.8 g/L | 1.7 g/L | 5 g/L |

(Reference example 12) Cadaverine fermentation

Corynebacterium glutamicum TR-CAD1 described in Japanese Unexamined Patent Publication (Kokai) No. 2004-222569 was used as microorganism for cadaverine production to study cadaverine fermentation, which consumes glucose. With respect to the culture medium to be used, a cadaverine fermentation culture medium was produced by preparing a glucose based carbon source having a content as shown in Table 7 and using a 3M aqueous ammonia to adjust the pH value to 7.0. The content of the cadaverine product was measured by HPLC and used for evaluation. Glucose Test Wako C (supplied by Wako Pure Chemical Industries, Ltd.) was used for measuring the glucose concentration.

Corynebacterium glutamicum TR-CAD1 was put in 5mL cadaverine fermentation culture medium prepared by adding kanamycin (25 μg/ml) in a test tube and shaken overnight to ensure culture (preculture). From the preculture solution, Corynebacterium glutamicum TR-CAD1 was recovered by centrifugal separation and rinsed adequately with 15 mL of sterilized water. The fungus body rinsed was put in 100 ml of the culture medium, and shaken for 24 hours in a 500 ml Sakaguchi flask to ensure culture (main culture).

[Table 7]

| constituent | Content of constituent |
|---|---|
| glucose | 50 g/L |
| citric acid | 1 g/L |
| urea | 15 g/L |
| monobasic potassium phosphate | 0.5 g/L |
| dibasic potassium phosphate | 0.5 g/L |
| magnesium sulfate heptahydrate | 0.5 g/L |
| L-threonine | 0.8 g/L |
| L-methionine | 0.6 g/L |
| L-leucine | 1.5 g/L |
| iron sulfate heptahydrate | 6.0m g/L |
| manganese sulfate monohydrate | 4.2m g/L |
| biotin | 1.0m g/L |
| thiamine | 2.0m g/L |

(Reference example 13) D-lactic acid fermentation

NBRC10505 pTM63 yeast as described in Japanese Unexamined Patent Publication (Kokai) No. 2007-074939 was used as microorganism while a D-lactic acid production culture medium with a composition as given in Table 8 was used as culture medium to produce a D-lactic acid, followed by determining its content by HPLC. Glucose Test Wako C (supplied by Wako Pure Chemical Industries, Ltd.) was used for measuring the glucose concentration.

NBRC10505/pTM63 strain was put in a 5 ml D-lactic acid production medium in a test tube and shaken overnight for culture (preculture). The resulting culture solution was put in a 50 ml fresh D-lactic acid production medium, and shaken in a 500 ml Sakaguchi flask at a temperature of 30°C for 24 hours to ensure culture (main culture).

[Table 8]

| constituent | Content of constituent |
|---|---|
| glucose | 50 g/L |
| yeast nitrogen base w/o amino acid | 6.7 g/L |
| standard 19 amino acids excluding leucine | 152 mg/L |
| leucine | 760 mg/L |
| inositol | 152 mg/L |
| p-aminobenzoic acid | 16 mg/L |
| adenine | 40 mg/L |

(Reference example 14) Succinic acid fermentation

As a microorganism for producing succinic acid, Anaerobiospirillum succiniciproducens ATCC53488 was used to perform succinic acid fermentation. A 100 mL seed culture medium with a composition as given in Table 9 was put in a 125 mL Erlenmeyer flask and heat-sterilized.
In an anaerobic glove box, 1 mL of 30 mM $Na_2CO_3$ and 0.15 mL of 180 mM $H_2SO_4$ were added, and furthermore, 0.5 mL of a reducing solution composed of 0.25 g/L cysteine. HCl and 0.25 g/L $Na_2S$ was added, followed by inoculation of ATCC53488 strain and culture at 39°C for 24 hours (main culture).
[Table 9]

| constituent | content of constituent |
|---|---|
| glucose | 50 g/L |
| polypeptone | 10 g/L |
| yeast extract | 5 g/L |
| dibasic potassium phosphate | 1 g/L |
| sodium chloride | 1 g/L |
| magnesium chloride | 0.2 g/L |

(Example 10)

One litter of the aqueous sugar solution resulting from step (1) in Example 1 and 1 liter of the aqueous sugar solution resulting from nanofiltration in Example 9 were put in a rotary evaporator (supplied by Tokyo Rika) to concentrate them about 3-fold by evaporating water under reduced pressure (200 hPa). The resulting material and a reagent glucose, adopted for comparison, were used for main culture in culture mediums prepared so as to suit for fermentation under the concentration conditions for each culture medium component under the fermentation conditions given in Reference examples 10 to 14. Here, reagent monosaccharides were used for preculture, and the sugar solutions were used only in main culture.
As a result, impediment to fermentation was depressed and the accumulated concentration was improved in solutions treated in steps (2) to (3) and a subsequent nanofiltration step as compared to untreated ones obtained from step (1) in Example 1, as seen from Table 10.
[Table 10]

| | sugar solution | refined sugar solution | reagent monosaccharide |
|---|---|---|---|
| L-lactic acid (Reference example 10) | 4 g/L | 8 g/L | 9 g/L |
| ethanol (Reference example 11) | 20 g/L | 28 g/L | 28 g/L |

(continued)

| | sugar solution | refined sugar solution | reagent monosaccharide |
|---|---|---|---|
| cadaverine (Reference example 12) | 0.3 g/L | 1.1 g/L | 1.3 g/L |
| D-lactic acid (Reference example 13) | 1 g/L | 7 g/L | 9 g/L |
| succinic acid (Reference example 14) | 28 g/L | 35 g/L | 35 g/L |

[Industrial applicability]

The present invention makes it possible to remove biomass residues from an aqueous sugar solution derived from cellulose-containing biomass, in the course of production of an aqueous refined sugar solution, so that clogging of the filters used for microfiltration and/or ultrafiltration can be prevented, thereby serving for fermentative production of various chemical products with increased efficiency by using this aqueous refined sugar solution as fermentation feedstock.

Explanation of numerals

1. raw water tank
2. cell having a filter for nanofiltration or reverse osmosis
3. high pressure pump
4. flow of membrane-permeated liquid
5. flow of membrane-concentrated liquid
6. flow of nanofiltrated liquid or culture solution supplied by high pressure pump

## Claims

**1.** An aqueous refined sugar solution production method using cellulose-containing biomass as feed material comprising:

(1) a step of decomposing cellulose-containing biomass to produce an aqueous sugar solution,
(2) a step of subjecting the aqueous sugar solution resulting from step (1) to coagulation treatment with a coagulant to produce an aqueous sugar solution with an average particle diameter of 2 μm or more as determined by particle size distribution measurement.
(3) a step of subjecting the aqueous sugar solution resulting from step (2) to microfiltration and/or ultrafiltration to recover an aqueous sugar solution from the downstream side,

**2.** An aqueous refined sugar solution production method as defined in claim 1 further comprising a step of subjecting the aqueous sugar solution resulting from step (3) to nanofiltration and/or reverse osmosis treatment to recover an aqueous refined sugar solution from the upstream side while removing fermentation impeding substances from the downstream side.

**3.** An aqueous refined sugar solution production method as described in either claim 1 or 2 wherein a cationic polymer coagulant is used for coagulation treatment in step (2).

**4.** An aqueous refined sugar solution production method as described in any of Claims 1 to 3 wherein an inorganic coagulant and an organic polymer coagulant are used in combination for coagulation treatment in step (2).

**5.** An aqueous refined sugar solution production method as described in any of claims 1 to 4 wherein coagulation treatment is performed a plurality of times repeatedly in step (2).

**6.** An aqueous refined sugar solution production method as described in claim 2 wherein said fermentation impeding substances include one or more selected from the group consisting of an organic acid, a furan based compound, and a phenolic compound.

7. An aqueous refined sugar solution production method as described in claim 6 wherein said organic acid is either formic acid or acetic acid.

8. An aqueous refined sugar solution production method as described in claim 6 wherein said furan based compound is either hydroxymethyl furfural or furfural.

9. An aqueous refined sugar solution production method as described in claim 6 wherein said phenolic compound is vanillin, acetovanillin, or syringic acid.

10. An aqueous refined sugar solution production method as described in any of claims 1 to 9 wherein the aqueous sugar solution resulting from step (2) is an aqueous sugar solution containing monosaccharides as main component.

11. An aqueous refined sugar solution production method as described in claim 2 wherein the step of subjecting the aqueous sugar solution resulting from step (3) to nanofiltration and/or reverse osmosis treatment is carried out by first filtering the aqueous sugar solution through a nanofiltration membrane and subsequently filtering the resulting filtrate through a reverse osmosis membrane.

12. An aqueous refined sugar solution production method as described in claim 2 wherein functional layers in the filters used for nanofiltration and/or reverse osmosis treatment are formed of polyamide.

13. An aqueous refined sugar solution production method as described in claim 2 wherein the functional layer in the filter used for nanofiltration is formed mainly of crosslinked piperazine polyamide and contains a component as represented by chemical formula 1.

[Chemical formula 1]

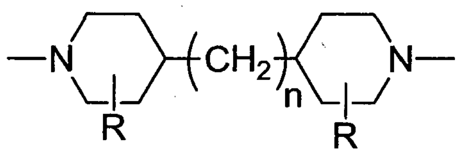

(where R represents -H or- $CH_3$, and n represents an integer of 0 to 3).)

14. A chemical product production method wherein an aqueous refined, sugar solution produced by an aqueous refined sugar solution production method as described in any of claims 1 to 13 is used as fermentation feedstock.

Figure 1

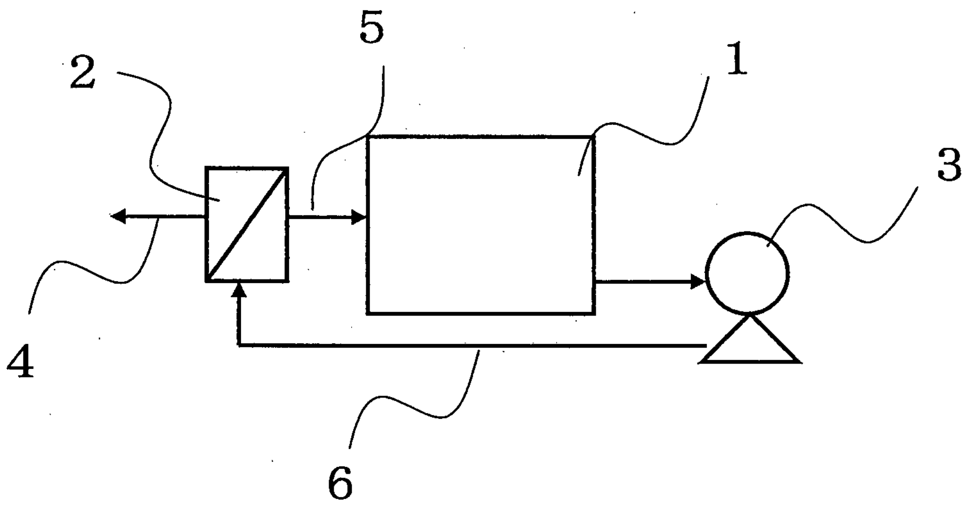

## INTERNATIONAL SEARCH REPORT

International application No.
PCT/JP2011/058963

A. CLASSIFICATION OF SUBJECT MATTER
*C12P19/02*(2006.01)i, *C13K1/02*(2006.01)i, *C07H3/02*(2006.01)n

According to International Patent Classification (IPC) or to both national classification and IPC

B. FIELDS SEARCHED

Minimum documentation searched (classification system followed by classification symbols)
C12P19/02, C13K1/02, C07H3/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2009-240167 A (Honda Motor Co., Ltd.), 22 October 2009 (22.10.2009), entire text (Family: none) | 1-14 |
| Y | JP 2009-501524 A (The Texas A & M University System), 22 January 2009 (22.01.2009), paragraphs [0013] to [0036] & US 2007/0014895 A1 & EP 1902139 A & WO 2007/009085 A2 | 1-14 |
| Y | WO 2009/110374 A1 (Toray Industries, Inc.), 11 September 2009 (11.09.2009), abstract; examples & EP 2251427 A1 & AU 2009222310 A & CA 2717298 A | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 July, 2011 (01.07.11) | 12 July, 2011 (12.07.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2011/058963

C (Continuation). DOCUMENTS CONSIDERED TO BE RELEVANT

| Category* | Citation of document, with indication, where appropriate, of the relevant passages | Relevant to claim No. |
|---|---|---|
| Y | JING JI.et al., Influence of organic and inorganic flocculants on physical-chemical properties of biomass and membrane-fouling rate, Water Research, 2010.03, Vol.44, p.1627-1635 | 1-14 |
| Y | GUIGUI C.et al., Combination of coagulation and ultrafiltration for drinking water production: impact of process configuration and module design., Water Sci Technol Water Supply, 2001, Vol.1, p.107-118 | 1-14 |
| Y | JP 2004-255368 A (Toray Industries, Inc.), 16 September 2004 (16.09.2004), paragraph [0036] (Family: none) | 1-14 |
| P,Y | DUARTE G.V.et al., Polymer induced flocculation and separation of particulates from extracts of lignocellulosic materials, Bioresource Technology, 2010.11, Vol.101, p.8526-8534 | 1-14 |
| A | JP 58-98100 A (The Regents of the University of California), 10 June 1983 (10.06.1983), entire text & US 4384897 A & CA 1183790 A | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI11506934 B
- JP 2005229821 A
- JP 2003212888 A
- JP 2001095597 A
- JP 3041380 B
- JP 2006088136 A
- JP 2009240167 A
- JP SHO62201606 B
- WO 2007097260 A
- JP 2007074939 A


### Non-patent literature cited in the description


- **A. ADEN et al.** Lignocellulosic Biomass to Ethanol Process Design and Economics Utilizing Co-Current Dilute Acid Prehydrolysis and Enzymatic Hydrolysis for Corn Stover. *NREL Technical Report,* 2002
- Hymenological Experiments Series Volume III Artificial Membrane. Kyoritsu Shuppan Co., Ltd, 1993, vol. III, 92